(19) Europäisches Patentamt
European Patent Office
Office européen des brevets

(11) EP 1 326 177 A1

(12) **EUROPEAN PATENT APPLICATION**
published in accordance with Art. 158(3) EPC

(43) Date of publication:
**09.07.2003 Bulletin 2003/28**

(21) Application number: **01965563.8**

(22) Date of filing: **10.09.2001**

(51) Int Cl.7: **G06F 17/30**, G06F 17/60

(86) International application number:
**PCT/JP01/07830**

(87) International publication number:
**WO 02/023395 (21.03.2002 Gazette 2002/12)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **12.09.2000 JP 2000276699**

(71) Applicant: **Institute of Medicinal Molecular Design, Inc.**
**Tokyo 113-0033 (JP)**

(72) Inventor: **ITAI, Akiko**
**Bunkyo-ku, Tokyo 113-0033 (JP)**

(74) Representative:
**Sternagel, Fleischer, Godemeyer & Partner**
**Patentanwälte**
**An den Gärten 7**
**51491 Overath (DE)**

# (54) METHOD OF FORMING MOLECULAR FUNCTION NETWORK

(57) A method of generating a molecule-function network including bio-events by carrying out a connect search using a biomolecule-linkage database including information on the bio-events, and a method of predicting a pathway between an arbitrary biomolecule and an arbitrary bio-event in said network or a method of predicting the bio-events to which an arbitrary biomolecule in said network is related.

Fig.1

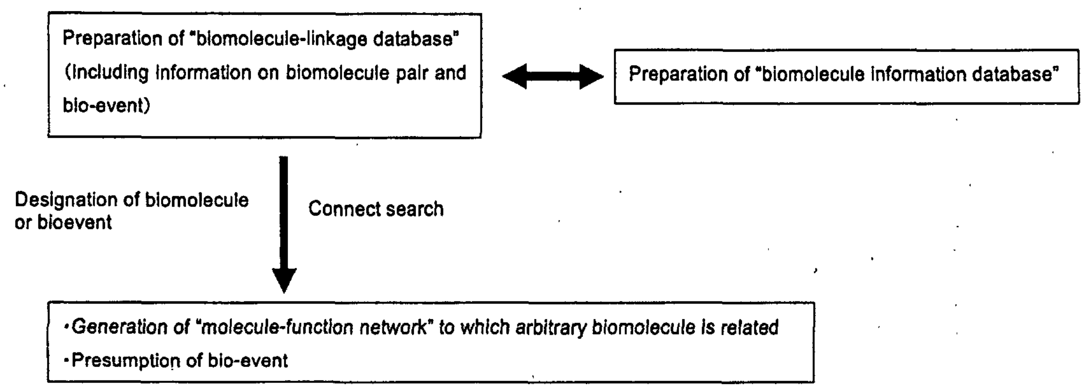


EP 1 326 177 A1

## Description

Technical Field

The present invention relates to a generation method and use of a biomolecule database including bio-event information.

Background Art

In an organism, various molecules such as amino acids, nucleic acids, lipids, carbohydrates and general small molecules as well as biomolecules such as DNA, RNA, proteins and polysaccharides exist, and each bears its function. Characteristics of a biological system are not only that it is constituted of various biomolecules, but also that all phenomena in an organism such as an expression of a function occur through a specific binding between biomolecules. In this specific binding, a covalent bond is not formed, instead, a stable complex is formed by an intermolecular force. Therefore, a biomolecule exists in equilibrium between an isolated state and a complex state, and between certain biomolecules, a stability of a complex state is greater and the equilibrium is remarkably biased to a complex side. As a result, in the existence of many other molecules, a molecule can distinguish and bind to a specific partner practically even in a fairly diluted concentration. In enzyme reactions, a substrate is released as a reaction product after receiving a specific chemical conversion in a complex state with an enzyme, and in signal transduction, an extracellular signal is transmitted into a cell through a structural change of a target biomolecule which occurs upon binding of a mediator molecule to the target biomolecule.

Recently, a progress of genome study is remarkable, genome sequences of various species including human have been elucidated, and genome-wide systematic studies are underway for genes and sequences of proteins which are the products of genes, expression of proteins in each organ, protein-protein interactions and others. Most of the results of these studies are open to public as databases, and are available for use throughout the world. Elucidation is progressing little by little regarding functions of genes and proteins, prediction of a gene which causes or is a background of a disease, and a relation with gene polymorphism, consequently, expectation for a medical treatment and a drug development based on genetic information is increasing.

On the other hand, whereas a bearer of genetic information is the nucleic acid, most biological functions such as energy metabolism, substance conversion and signal transduction are born by molecules other than a nucleic acid. A protein is different from molecules of other categories in a point where it is directly produced based on a design chart called gene, and there are many kinds of proteins. Enzymes, target biomolecules of a small-molecular intrinsic physiologically active compound, target biomolecules (modified with sugar in many cases) of an intrinsic physiologically-active protein are all proteins. Set a primary cause of a disease aside, it is considered that many diseases and symptoms are results of abnormality of amount or balance of a protein or a small molecule, or in some cases, quality (function) of those molecules. Most of the existing drugs are compounds that act to a protein as a target and control its functions. Different from a protein, there is a reason in which a steric structure of a nucleic acid has a difficulty to demonstrate its specificity as a target of a small molecular drug, nonetheless, targets of antibiotics and antibacterial agents as well as agrochemicals such as insecticides and antimycotic agents are proteins.

Therefore, in order to carry out medical treatment or drug development based on genetic information, it is necessary to clarify a function of each protein and a small molecule in an organism and a specific relation between those molecules. Furthermore, since different enzymes play their parts one after another in biosynthesis of a necessary molecule and since different molecules bind together in turn in signal transduction, these molecules have direct or indirect, functional or biosynthetic, mutual linkage, hence information on the linkage (molecule-function network) is important. Moreover, with the studies so far, many molecules such as mediators and hormones which directly involve in occurrences of various clinical symptoms, physiological phenomena, and biological responses have been discovered, and it is inevitable for an appropriate treatment to correlate those molecules with a molecule-function network. On the other hand, in a strategy for drug development, it is necessary to take account of a molecule-function network including target molecules, in order to select an appropriate target molecule for drug development while considering a risk of side effects.

As databases related to proteins, SwissProt (the Swiss Institute of Bioinformatics (SIB), European Bioinformatics Institute (EBI)) and PIR (National Biomedical Research foundation (NBRF)) are known, and both contain annotation information on species, function, functional mechanism, discoverer, literature and others as well as sequence information.

Among molecule-network databases focusing on the linkage of molecules, KEGG (Kanehisa et al., Kyoto University), Biochemical Pathways (Boehringer Mannheim), WIT (Russian Academy of Sciences), Biofrontier (Kureha Chemical Industry), Protein Pathway (AxCell), bioSCOUT (LION), EcoCyc (DoubleTwist), and UM-BBD (Minnesota Univ.) are known as databases about metabolic pathways.

The PATHWAY database of KEGG contains metabolic pathways and signal transduction pathways, wherein the former treats metabolic pathways of general small molecules involved in substance metabolism and energy metabolism, and the latter treats proteins of signal transduction system. In both, pre-defined molecule networks are provided as static Gif files. In the former, information on enzymes and ligands is imported from separate text-style molecule databases, LIGAND (Kanehisa et al., Kyoto Univ.) and ENZYME (IUPAC-IUBMB). Information on enzymes involved in syntheses of physiologically active peptides and information on target biomolecules are not included.

EcoCyc is a database of substance metabolism in Escherichia coli, and it displays a pathway diagrammatically based on data about individual enzyme reactions and data about known pathways (represented as a collection of enzyme reactions belonging to said pathway). As a search function of EcoCyc, search by a character string or an abbreviated symbol for a molecule name or a pathway name is provided, however, it is not possible to search a new pathway by specifying an arbitrary molecule.

Those concerning signal transduction, CSNDB (National Institute of Health Sciences, Japan), SPAD (Kuhara et al., Kyushu Univ.), Gene Net (Institute of Cytology & Genetics Novosibirsk, Russia), and GeNet (Maria G. Samsonova) are known.

As databases of protein-protein interaction, DIP (UCLA), PathCalling (CuraGen), and ProNet (Myriad) are known.

As databases of expressions of gene or protein, BodyMap (Univ. of Tokyo and Osaka Univ.), SWISS-2DPAGE (Swiss Institute of Bioinformatics), Human and mouse 2D PAGE database (Danish Centre for Human Genome Research), HEART-2DPAGE (GermanHeart), PDD Protein Disease Databases (NIMH-NCI), Washington University Inner Ear Protein Database (Washington Univ.), PMMA-2DPAGE (Purkyne Military Medical Academy), Mito-Pick (CEA, France), Molecular Anatomy Laboratory (Indiana University), and Human Colon Carcinoma Protein Database (Ludwig Institute for Cancer Research) are known.

As examples of molecule network for biological response simulation, E-Cell (Tomita et al., Keio Univ.), e E.coli (B. Palsson), Cell (D. Lauffenburger, MIT), Virtual Cell (L. Leow, Connecticut Univ.), and Virtual Patient (Entelos, Inc.) are known.

Concerning relations between biomolecules and functions, SwissProt collects broad information on protein, and COPE (University of Munich) provides information on functions of cytokines in a text format. ARIS (Japan Information Processing Service Co. Ltd.) records literature information on side effects and interactions of drugs and on toxication by agrochemicals and chemicals gathered from approximately 400 domestic journals and 20 foreign journals mostly on medical and pharmacological fields, however, a database for physiological actions and responses above cellular level of biomolecules are not available so far. Concerning genes and diseases, OMIM (NIH) collects information on genetic diseases and amino acid mutations of proteins. The data is described in a text format and can be searched by keyword.

A problem of the existing databases focusing on linkages between molecules is as follows. Molecule-network databases have been prepared for systems in which molecules included and linkages between the molecules are known, and since it is possible to arrange molecules beforehand considering the relation between the molecules, static representation such as Gif has been sufficient. However, with such a method, it is difficult to add new molecules and linkages between the molecules. There exist more than 100,000 molecules including molecules that will be revealed in the future (the number of molecules that KEGG treats is about 10,000 including drug molecules), and when the linkages between those molecules will be elucidated in the future research, it is expected that the complexity of the molecule network will increase acceleratingly. We need a new method that is well adapted to additions of new molecules, and can generate a partial molecule network containing necessary information while retaining information on huge number of molecules and relations between the molecules.

As of Sept. 7, 2001, KEGG stores linkages between molecules as information on pairs of two molecules, and it is possible to search for a pathway which links arbitrary two molecules in metabolic pathways using that information. However, pathway search problem like this has difficulty that the longer the pathway linking the two molecules, the exponentially more the computation time.

On the other hand, there is no limit to additions of molecule data in a text database. However, it is difficult to generate a molecule network representing linkages of many molecules by repeating searches one after another for functionally or biosynthetically related molecules from a data of each molecule. It is necessary to develop methods of storing and searching data so that linkages for necessary molecules are obtained dynamically and automatically at the time of the search. Furthermore, in order to understand diseases and pathological states at molecular level, we need a new invention to describe relations between biomolecule / molecule network and biological responses / physiological actions.

## Disclosure of Invention

An object of the present invention is to provide schemes and methods to understand various biological responses and phenomena in the light of the functions of biomolecules and relations between those molecules, and to be more specific, to provide databases and search methods that can link information on biomolecules to biological responses. Furthermore, one of the other objects of the present invention is to provide a method of extracting rapidly and efficiently, from the huge amount of information, only signal transduction pathways and biosynthetic pathways related to an arbitrary biological response or biomolecule, and predicting a promising drug target and a risk of side effects.

As a result of zealous endeavor to solve the aforementioned object, the inventors found that the aforementioned object can be solved by covering linkages between biomolecules by accumulating information wherein a pair of direct-binding biomolecules is taken as a part, by attaching bio-event information comprising physiological actions, biological responses, clinical symptoms and others to a pair between a key molecule involved directly in the expression of a biological response and its target biomolecule, and by generating a molecule-function network by searching linkages automatically one after another which include designated one or more arbitrary biomolecules or bio-events.

That is, the present invention provides a method of generating a molecule-function network by using a biomolecule-linkage database that accumulates information on direct-binding biomolecule pairs. In preferred embodiments of this invention, there are provided the aforementioned method which generates a molecule-function network related with bio-event information by using biomolecule-linkage database comprising bio-event information; the aforementioned method which uses a biomolecule-information database comprising information on biomolecules themselves; and the aforementioned method which generates a molecule-function network including drug molecules related with bio-event information. Furthermore, the present invention also provides a method of predicting bio-events directly or indirectly related to an arbitrary biomolecule or a drug molecule by using a biomolecule-linkage database which accumulates information on bio-events concerning a direct-binding biomolecule. Moreover, the present invention provides a method of analyzing information on polymorphism or expression of genes using a molecule-function network, by generating a database which links a molecule ID of a biomolecule with a name, an ID, or an abbreviated name of a gene when the biomolecule is a protein coded by the gene in an external database or a literature.

In more preferred embodiments of the present invention, there are provided the aforementioned method characterized by hierarchizing the molecule-function network based on the belonging subnet and inclusion relationships among subnets wherein biomolecule pairs grouped based on the linkage on the network are treated as a subnet; the aforementioned method characterized by hierarchical storage of information on biomolecule pairs based on belonging pathway name, belonging subnet name and others; the aforementioned method characterized by hierarchical storage of information on biomolecules themselves based on expression patterns from genes and expression patterns on cell surface and others; and the aforementioned method characterized by hierarchical storage of information on bio-events based on classification by the superordinate concept of said event and/or based on the relation with pathological events. Furthermore, there are also provided by the present invention, the aforementioned method characterized by storage of information on relationship and dependence among stored items at upper hierarchy comprising upper hierarchy of biomolecule pairs, upper hierarchy of biomolecules themselves and upper hierarchy of bio-events; the aforementioned method characterized by facilitating generation of a molecule-function, network using hierarchical information stored in a biomolecule information database or a biomolecule-linkage database; and the aforementioned method characterized by controlling the details in representation of a molecule-function network using hierarchical information stored in a biomolecule information database or biomolecule-linkage database.

Moreover, by the present invention, the following methods and databases are provided.

1. A method of relating information on bio-events with biomolecules.
2. A method of generating a molecule-function network related with information on bio-events.
3. A method of generating a molecule-function network including drug molecules related with information on bio-events.
4. A method of predicting bio-events with which an arbitrary biomolecule relates directly or indirectly.
5. A method of predicting bio-events with which an arbitrary biomolecule relates directly or indirectly using a biomolecule-linkage database having information on bio-events.
6. A method of predicting a molecule-function network with which an arbitrary biomolecule relates and bio-events with which said molecule relates directly or indirectly using a biomolecule-linkage database having information on bio-events.
7. A biomolecule-linkage database wherein pairs of key molecules directly involved in expression of bio-events and their target biomolecules and information on said bio-events are added to information on pairs of direct-binding biomolecules.
8. A biomolecule-linkage database comprising information on bio-events arisen from key molecules.
9. A biomolecule-linkage database comprising key molecules having information on bio-events.
10. A molecule-function network obtained by a connect search of a biomolecule-linkage database.
11. A method of predicting a molecule-function network and bio-events with which an arbitrary biomolecule is related using one of the aforementioned biomolecule-linkage database described in 7 through 9.
12. A method of predicting a molecule-function network and bio-events with which an arbitrary biomolecule or a drug molecule is related using one of the aforementioned biomolecule-linkage databases described in 7 through 9 and a drug molecule-linkage database.
13. The method or the biomolecule-linkage database or the molecule-function network described in the aforementioned 1 through 12, wherein the information on bio-events comprises up-or-down information corresponding to quantitative or qualitative changes of key molecules.

14. The method or the biomolecule-linkage database or the molecule-function network described in the aforementioned 1 through 12, wherein the information on bio-events comprises information on originating organs of the key molecule and expressing organs of the bio-event.

15. The method or the biomolecule-linkage database or the molecule-function network described in the aforementioned 1 through 12, wherein the information on bio-events comprises up-or-down information corresponding to quantitative or qualitative changes of the key molecule and information on originating organs of the key molecules and expressing organs of the bio-events.

16. A method of generating a molecule-function network with which one or more arbitrary biomolecules relate directly or indirectly, functionally or biosynthetically, by storing information describing pairs of direct-binding biomolecules and the relation of said binding.

17. A method of searching key molecules that relate directly or indirectly with an arbitrary biomolecule functionally or biosynthetically using a collection of information on pairs of direct-binding biomolecules.

18. A method of predicting bio-events with which an arbitrary biomolecule relates directly or indirectly based on the method described in 17.

19. A method of generating a molecule-function network that indicates functional or biosynthetic relation between biomolecules by storing information describing pairs of direct-binding biomolecules and the relation of said binding.

20. A method of generating a molecule-function network related to one or more arbitrary biomolecules by storing information describing pairs of direct-binding biomolecules and the relation of said binding as parts, and by carrying out a connect search.

21. A method of extracting a group of biomolecules which relate directly or indirectly with one or more designated biomolecules biosynthetically or functionally by storing information describing pairs of direct-binding biomolecules and the relation of said binding as parts, and by carrying out a connect search.

22. A method of predicting a disease-related molecule-function network based on a group of bio-events related to said disease.

23. A method of predicting a disease-related molecule-function network and predicting a possible drug target, based on a group of bio-events related to said disease.

24. A method of predicting a risk of side effects when a biomolecule on a disease-related molecule-function network is selected as a drug target, based on a group of bio-events related to said disease.

25. A method of predicting up-or-down of bio-events by a control of the function of an arbitrary biomolecule on a disease-related molecule-function network.

26. A method of supporting the selection of a drug target using information on quantitative changes of key molecules and up-or-down of bio-events.

27. A biomolecule-linkage database to be used in the method described in the aforementioned 26.

28. A biomolecule-linkage database comprising information on pairs of a drug molecule and its target biomolecule.

29. A biomolecule-linkage database comprising information on pairs of a drug molecule and its target biomolecule and information on actions and side effects.

30. A method of predicting or avoiding a risk of side effects of a drug molecule or an interaction between drugs using a biomolecule-linkage database comprising information on pairs of a drug molecule and its target biomolecule and information on actions and side effects.

31. A method of selecting a drug compound and determining a dose for a medical treatment using a biomolecule-linkage database comprising information on pairs of a drug molecule and its target biomolecule and information on actions and side effects, and by linking to the information on gene polymorphism as necessary.

32. The method or the biomolecule-linkage database or the molecule-function network described in the aforementioned 1 through 31 characterized in that the proteins in the biomolecule-linkage database or the molecule-function network are linked to a gene database.

33. The method or the biomolecule-linkage database or the molecule-function network described in the aforementioned 1 through 31 characterized in that the biomolecule-linkage database or the molecule-function network is linked to the information on genes corresponded with genomic sequences.

34. The method or the biomolecule-linkage database or the molecule-function network described in the aforementioned 1 through 31 characterized in that the biomolecule-linkage database or the molecule-function network is linked to the information on genes corresponded with information on protein expression in organs.

35. The method or the biomolecule-linkage database or the molecule-function network described in the aforementioned 1 through 31 characterized in that the biomolecule-linkage database or the molecule-function network is linked to the information on genes involved in gene polymorphism.

36. The method or the biomolecule-linkage database or the molecule-function network described in the aforementioned 1 through 31 characterized in that the biomolecule-linkage database or the molecule-function network is linked to the information on genome or genes corresponded with genome or gene sequences of other species.

37. The method or the biomolecule-linkage database or the molecule-function network described in the aforementioned 1 through 31 for predicting a mechanism of a disease using the information on changes in protein expression in specific organs upon administration of a drug molecule.

38. The method or the biomolecule-linkage database or the molecule-function network described in the aforementioned 1 through 31 to be used to analyze the information on a group of gene polymorphism observed with high frequency in a specific disease.

39. The method or the biomolecule-linkage database or the molecule-function network described in the aforementioned 16 through 21 characterized in that the relation of a biomolecule pair is categorized.

40. The method or the biomolecule-linkage database or the molecule-function network described in the aforementioned 1 through 31 characterized in that the bio-event is categorized.

41. The method or the biomolecule-linkage database or the molecule-function network described in the aforementioned 13 through 15 characterized in that the information on up-or-down of the bio-event upon a quantitative change of the key molecule is categorized.

42. The method or the biomolecule-linkage database or the molecule-function network described in the aforementioned 1 through 41 characterized in that two or more biomolecules are treated as one virtual biomolecule as necessary.

43. The method or the biomolecule-linkage database or the molecule-function network described in the aforementioned 1 through 41 characterized in that one or more distributed biomolecule-linkage databases are used via communication.

44. The method or the biomolecule-linkage database or the molecule-function network described in the aforementioned 1 through 41 characterized in that a database containing the information on biomolecules directly involved in expressions of bio-events is prepared and used with a database of molecule-function networks that does not necessarily contain information on bio-events.

45. The method or the biomolecule-linkage database or the molecule-function network described in the aforementioned 1 through 41 characterized in that a partial molecule-function network related to an arbitrary molecule is extracted from a database of molecule-function networks that does not necessarily contain information on bio-events, and a database containing the information on biomolecules directly involved in expressions of bio-events is searched based on the molecules constituting said network.

46. A biomolecule-linkage database wherein the biomolecule or biomolecule pairs to be treated are screened based on the information on originating organs or acting organs and others, or a molecule-function network generated using that database, or a method of generating a molecule-function network using that database.

47. A method of further screening of molecule-function networks, that are generated by a connect search of a biomolecule-function database beforehand, based on the information on biomolecules or bio-events or others included in each network, or molecule-function networks generated by the further screening.

48. A method of further screening of molecule-function networks, that are generated using a biomolecule-linkage database wherein the biomolecule or biomolecule pairs to be treated are screened based on the information on originating organs or acting organs and others, based on the information on biomolecules or bio-events or others included in each network, or molecule-function networks generated by the further screening.

49. A computer system comprising programs and databases for carrying out the methods described in the aforementioned 1 through 48.

50. A computer-readable medium recording the databases described in the aforementioned 1 through 48.

51. A computer-readable medium recording information on the molecule-function network described in the aforementioned 1 through 48.

52. A computer-readable media recording the databases described in the aforementioned 1 through 48 and programs for carrying out the methods described in the aforementioned 1 through 48.

53. A method of correlating information on hierarchized bio-events with biomolecules.

54. A method of generating a molecule-function network correlated with hierarchized bio-events.

55. A method of generating a molecule-function network characterized by hierarchical storage of information on pairs of biomolecules.

56. A method of generating a molecule-function network characterized by hierarchical storage of complexation states of biomolecules.

57. A method of correlating bio-events to hierarchically-stored information on biomolecule pairs.

58. A method of correlating bio-events to hierarchically-stored information on complexation states of biomolecules.

59. A method of generating a molecule-function network characterized by hierarchical storage of information on transcription of a group of genes.

60. A method of generating a molecule-function network characterized by hierarchical storage of information on protein expression.

61. A method of generating a molecule-function network based on the search result obtained by carrying out a search based on keyword and/or numerical parameter and/or molecular structure and/or amino acid sequence and/or base sequence and/or others to arbitrary data items in the database.

62. A method of obtaining a subset of said molecule function network by carrying out a search based on keyword and/or numerical parameter and/or molecular structure and/or amino acid sequence and/or base sequence and/or others to the data on biomolecules and/or biomolecule pairs and/or bio-events included in a generated molecule-function network.

63. A method of highlighting the biomolecules and/or the biomolecule pairs and/or the bio-events by carrying out a search based on keyword and/or numerical parameter and/or molecular structure and/or amino acid sequence and/or base sequence and/or others to the data on biomolecules and/or biomolecule pairs and/or bio-events included in a generated molecule-function network.

Brief Description of the Drawings

Figure 1 shows a basic concept of the method of the present invention.

Figure 2 shows a concept when a drug molecule-linkage database is used in the method of the present invention.

Figure 3 shows a concept when a genetic information database is used in the method of the present invention.

Figure 4 shows a concept of the renin-angiotensin system which is treated in Example 1.

Figure 5 shows contents of the biomolecule information database of Example 1.

Figure 6 shows contents of the biomolecule-linkage database of Example 1.

Figure 7 shows a molecule-function network obtained by a search about biomolecules in Example 1. The biomolecule and the bio-event used as a query are indicated in bold frames.

Figure 8 shows contents of the drug molecule information database in Example 1.

Figure 9 shows contents of the drug molecule-linkage database in Example 1.

Figure 10 shows a molecule-function network obtained by a search about a drug molecule in Example 1. The drug molecule and the bio-event used as a query are indicated in bold frames.

Figure 11 is a flow chart of the program for searching and displaying the molecule-function network in Example 2.

Figure 12 shows input items of the connect search (one point is designated) in Example 2.

Figure 13 shows input items of the connect search (two points are designated) in Example 2.

Detailed Description of the Preferred Embodiments

Meanings or definitions of the terms in the present description are as follows.

"Organism" is a concept including, for example, organelle, cell, tissue, organ, individual, a group of individuals, as well as parasite.

"Bio-event" is a concept including all phenomena, responses, reactions, and symptoms appearing endogenously or exogenously in an organism. Transcription, cell migration, cell adhesion, cell division, neural excitation, vasoconstriction, increase of blood pressure, decrease of blood glucose level, fever, convulsion, infection by a parasite such as a heterogeneous organism and a virus can be pointed out as specific examples. Furthermore, responses to physical stimulations such as light and heat from outside of an organism may be included in the concept of bio-event.

"Pathological event" is a concept that can be included in the "bio-event," and means a condition where a "bio-event" exceeds a certain threshold quantitatively or qualitatively, and can be judged as a disease or a pathological state. For example, as a consequence of an extraordinarily increased "bio-event" of blood pressure increase, high blood pressure or hypertension can be pointed out as the "pathological events", and when blood sugar is not controlled within a normal range, hyperglycemia or diabetes can be pointed out as the "pathological events". Moreover, there are pathological events that are related to multiple kinds of bio-events, as well as the aforementioned examples that are related to a single bio-event.

"Biomolecule" indicates organic molecules of various structures existing in an organism and groups of such molecules, such as nucleic acids, proteins, lipids, carbohydrates, general small molecules, and may contain metal ions, water, and a proton as well.

"Key molecule" mainly indicates molecules such as mediators, hormones, neurotransmitters and autacoids. In most cases, a specific target biomolecule exists in an organism, and it is known that a direct binding to that molecule acts as a trigger of the aforementioned "bio-event." Although these molecules are generated and exerting actions in an organism, a bio-event is generally expressed corresponding to the given amount even when they are given from outside of an organism. Adrenalin, angiotensin II, insulin, estrogen and others can be pointed out as specific examples.

"Target biomolecule" means a specific biomolecule that can accept a biomolecule such as a mediator, a hormone, a neurotransmitter, and an autacoid or a drug molecule. Direct binding to it causes expression of a specific event.

"Up-or-down information of a bio-event" is the information on exaltation / increase or supression / decrease in response to a quantitative or qualitative change of a key molecule or a target biomolecule. It includes a case where the bio-event occurs only after the amount of the key molecule exceeds a certain threshold.

"Molecule ID" is given for the purpose of identification or designation of a molecule instead of the molecule name, and needs to correspond to each molecule uniquely. An abbreviated symbol of a molecule name or an alphanumeric character string irrelevant to a molecule name may be acceptable, however, it is desirable to use a short character string. When there is a molecule ID that is already used globally, it is desirable to use it. It is possible to give multiple molecule IDs assigned by different methods to one molecule and to hierarchize them by structural group or function.

"Direct binding" means formation of a stable complex by an intermolecular force not by a covalent bond, or means possibility of complex formation. In rare cases, a covalent bond is formed, and such cases are included in this concept. It is also called "interaction", however, interaction includes broader meanings.

"Biomolecule pair" means a pair of biomolecules capable of direct binding or presumed to form direct binding in an organism. Estradiol and estrogen receptor, angiotensin converting enzyme and angiotensin I can be pointed out as specific examples. In a case of a molecule pair of an enzyme and a product in an enzyme reaction, its complex is not said to be very stable, however, it is regarded to be included in biomolecule pairs. Furthermore, as in the case of two protein molecules judged to have interaction by the tow-hybrid experimental technique, molecules pairs whose mutual roles are not clear may be included. For physical or chemical stimulations from outside of an organism such as light, sound, temperature change, magnetic field, gravity, pressure and vibration, these stimulations may be treated as virtual biomolecules, and a biomolecule pair to a corresponding target biomolecule may be defined.

"Structure code" is a classification code representing structural features whether a biomolecule is DNA, RNA, a protein, a peptide, or a general small molecule and others.

"Function code" is a classification code representing a function of a biomolecule at molecular level, for example, in the case of a biomolecule wherein the "structure code" is "protein", it represents a classification of membrane receptor / nuclear receptor / transporter / mediator / hydrolase / kinase / phosphorylase and others, and in the case of a biomolecule wherein the "structure code" is "small molecule", it represents a classification of substrate / product / precursor / active peptide / metabolite and others.

"Relation code" is a classification code representing a relation between two molecules constituting a biomolecule pair. It may be categorized, for example, 10 for an agonist and a receptor, 21 for an enzyme and a substrate, 22 for a substrate and a product. As in the case of two protein molecules considered to have an interaction by the two-hybrid experimental technique, when mutual role of two molecules is not clear, it is desirable to use a code representing such situation.

"Relation-function code" is a classification code representing a phenomenon or a change accompanied by a direct binding of two molecules constituting a biomolecule pair, and for example, a classification such as hydrolysis, phosphorylation, dephosphorylation, activation, inactivation may be used.

"Reliability code" is a code to indicate reliability level of the direct binding for each biomolecule pair and/or the experimental method whereupon the direct binding is proved.

"Connect search" means automatically searching a linkage of functionally or biosynthetically related molecules that include designated one or more arbitrary biomolecules or bio-events.

"Molecule-function network" means a linkage of functionally or biosynthetically related molecules obtained as a result of the connect search, by using a biomolecule-linkage database, wherein one or more arbitrary biomolecules or bio-events are designated.

"Drug molecule" means a molecule of a compound manufactured and used for medical treatment as a drug, and also includes a compound with known physiological activity such as a compound used for medical and/or pharmaceutical research and a compound described in patents or literatures.

"To correlate with information on bio-event" means to indicate or discover that the expression of a certain bio-event is related to a certain biomolecule, drug molecule, genetic information, or molecule-function network.

"Categorization" means classifying information on biomolecules, biomolecule pairs, bio-events and others into predetermined categories and describing said information with notations representing the pertinent categories, instead of storing the given information intact, when the information is stored into a database. The aforementioned examples in "structure code", "function code", "relation code", and "relation-function code" are the examples of "categorization".

"Originating organ" means organ, tissue, region in organ or tissue, specific cell in organ or tissue, region in cell and others, where a biomolecule is originated.

"Existing organ" means organ, tissue, region in organ or tissue, specific cell in organ or tissue, region in cell and others, where a biomolecule is stored after its generation.

"Acting organ" means organ, tissue, region in organ or tissue, specific cell in organ or tissue, region in cell and others, where a biomolecule or a key molecule causes a bio-event.

As one of the embodiments of the present invention, the following method is provided (Fig. 1). First, a "biomolecule-linkage database" storing the information on pairs of direct-binding biomolecules is prepared. Information on biomolecules themselves such as an assignment of a molecule ID to a biomolecule may be included here, however, it is desirable to store them in a separate database, a "biomolecule information database". Next, one or more arbitrary molecules are designated from the aforementioned "biomolecule-linkage database" and a connect search is carried out to obtain a "molecule-function network" which is a representation of the functional or biosynthetic linkage of one or more biomolecules.

By correlating information on bio-events to at least those biomolecule pairs consisting of a key molecule and its target biomolecule among biomolecule pairs, it is possible to presume, together with the "molecule-function network", bio-events to which molecules in the moleucle-function network are directly or indirectly related. Furthermore, by adding information on the relation between a quantitative or qualitative change of a key molecule and up-or-down of a bio-event, it is possible to presume whether a quantitative or qualitative change of an arbitrary molecule on the molecule-function network works for exaltation / increase of a bio-event or for suppression / decrease of a bio-event.

A principal role of the "biomolecule information database" is to define a molecule ID or an ID to the formal name of each biomolecule, and it is desirable to store necessary information on biomolecules themselves. For example, it is desirable to store information on molecule name, molecule ID, structure code, function code, species, originating organ, existing organ and others. Furthermore, even for a biomolecule that is not isolated experimentally nor confirmed to exist, one may assign a temporary molecule ID and other information, for example, to a molecule whose existence is predicted from experiments with other species.

Information on amino acid sequence and/or structure of each biomolecule may be included in the "biomolecule information database", however, it is desirable to store said information in a sequence database or a structure database and take out the information based on the molecule ID as necessary. For those with low molecular weight among biomolecules, it is desirable to store not only the formal molecule name but also the data necessary for drawing a chemical structure in the biomolecule information database or a separate database, so that chemical structures can be appended to the representation of the molecule-function network as necessary.

When it is more convenient to treat multiple biomolecules collectively, for example, two or more biomolecules showing activity or function in an oligomer or in a group, one may define them as one virtual biomolecule and register it in the "biomolecule information database" assigning a molecule ID. In this case, it is preferable to assign and register a molecule ID to each constituting molecule, and set up in the record of the virtual biomolecule, a field which describes molecule IDs of the constituting molecules, if the constituting molecules are known. Even when the constituting biomolecules are unknown, it is possible to define a virtual biomolecule having a specific function as a group, and use it for the definition of a biomolecule pair.

Furthermore, when a biomolecule consists of two or more domain structures, one may treat each domain as an independent molecule, if it is judged to be more favorable to treat each domain independently for those reasons such that the domains have different functions from each other. For example, it is preferable to give a molecule ID to each domain and register it in the biomolecule information database together with the original biomolecule. By setting up a field describing molecule IDs of the divided domains in the record of the original biomolecule, it is possible to describe that one biomolecule has two or more different functions. When a specific sequence on genome sequence which is not a gene has a certain function or is recognized by a specific biomolecule, it is possible to treat the part of the sequence as an independent biomolecule and assign a molecule ID for defining a biomolecule pair.

Information on the biomolecule pair is stored in the "biomolecule-linkage database." For each biomolecule pair, molecule IDs of two biomolecules forming the pair, relation code, relation-function code, reliability code, bio-events, acting organs, conjugating molecules, and other additional information are registered. For a molecule pair of a key molecule and its target biomolecule, it is desirable to input bio-events, up-or-down information of bio-events corresponding to a quantitative or qualitative change of either molecule, pathological events and others as much as possible. For a biomolecule pair without a key molecule, it is desirable to input bio-events and pathological events when there are bio-events or pathological events to which said biomolecule pair is directly related. Up-or-down information of a bio-event corresponding to a quantitative or qualitative change of a key molecule may be described as simplified information such that the bio-event increases or decreases compared to a normal range corresponding to the increase of the key molecule, for example. When one enzyme catalyses reactions of two or more kinds of substrates and generates different reaction products respectively, a representation specifying the relation among the enzyme, substrate and reaction product may be added.

Since the "biomolecule informaiton database" and the "biomolecule-lnkage database" are different in their contents and constitutions, they are treated as conceptually independent databases in the present description, however, it is needless to say that those two kinds of data may be stored in one database combining the both, in the light of the purpose of the present invention. Moreover, two or more "biomolecule information database" and two or more "biomolecule-linkage database" may exist, and in this case, it is possible to use those databases by selecting and combining them properly.

For example, data for different species distinguished by a specific field may be stored in the same "biomolecule information database" and "biomolecule-linkage database", or alternatively, data for human and mouse may be stored in separate databases.

As "relation code", one may input with words such that two molecules constituting a biomolecule pair are an agonist and a receptor, or an enzyme and a substrate, for example. However, it is desirable to input with categorization, for example, 10 for the relation between an agonist and a receptor, 21 for the relation between an enzyme and a substrate, 22 for the relation between an enzyme and a product. Furthermore, as "relation-function code", it is convenient to store the class of functions such as hydrolysis, phosphorization, dephosphorization, activation and inactivation, wherein it is desirable to input them with categorization.

Relations between biomolecule pairs are not always clear as in the case of an enzyme and a substrate. For example, like two protein molecules judged to have protein-protein interaction by the two-hybrid experimental technique, there are cases in which mutual roles of both molecules are not clear. In order to carry out a connect search including such biomolecule pairs, it is convenient to treat whether the relation between two molecules constituting the biomolecule pair is oriented or not. To each biomolecule pair, it is desirable to use a relation code that can distinguish to which case it belongs. The former case is treated as fixed acting direction and only the input order of the two molecules in the representation of the molecule pair is considered, whereas the latter case is treated as unknown acting direction and a relation with reverse direction is also considered at the time of search.

There are various kinds of information on directly-bonding biomolecule pairs, from definite information that have been experimentally proved, to those tentatively assumed as biomolecule pairs. Furthermore, in some experimental methods, there are cases that some biomolecule pairs are included by mistake due to false positives. Consequently, it is desirable to add "reliability code" to information on each biomolecule pair, which indicates the reliability level and the experimental method. When the molecule-function networks generated by a search are too large, it is possible to screen the network using this code.

If we retain information on the organs where a biomolecule is stored and information on the organs on which it is acting in addition to information on the organs where a biomolecule is generated, we can describe easily, at the time of the generation of a biomolecule-function network, such a phenomenon that a molecule generated in a certain organ and going outside a cell acts on the target biomolecule on the membrane of other cell from outside. It is desirable to input information on the originating organs and the existing organs of a biomolecule in the "biomolecule information database", and to input information on the acting organs in the "biomolecule-linkage database." Here, the description of the originating organs, existing organs, and acting organs is not particularly limited to organs, and may include information on tissue, region of organ or tissue, specific cell in organ or tissue, intracellular region and others.

Any descriptions are acceptable for describing the experimental or predictive method proving the direct binding, the kind of bio-event, up-or-down of a bio-event corresponding to a quantitative change of a key molecule, intracellular region, tissue, organ, region in organ, as long as they are simplified ones. However, it is desirable to categorize and convert them to short alphanumeric notations and others. If we define them in a dictionary of synonyms, we can process synonyms at the same time and minimize mistakes at the time of input.

A concept of the "connect search" which generates a "molecule-function network" from the "biomolecule-linkage database" is shown in the following. Any method may be used for the "connect search" of the present invention, as long as this concept is realized. For example, an algorithm of "depth first search" described in Chapter 29 of "Algorithm in C" (Addison-Wesley Pub Co, 1990) by Sedgewick may be used.

If we suppose that each biomolecule pair consisting of biomolecules represented by molecule IDs a~z is described as (n,m), a biomolecule-linkage database is described as a group of biomolecule pairs as follows. (a, c) (a, g) (b, f) (b, k) (c, j) (c, r) (d, v) (d, y) (e, k) (e, s) (g, u) (j, p) (k, t) (k, y) (p, q) (p, y) (x, z)

If we designate generation of a molecule-function network containing c and e, for example, in the connect search, biomolecule pairs (c, j) (j, p) (p, y) (y, k) (k, e) having one of the pair molecules in common are searched successively, and c - j - p - y - k - e which is a linkage of molecules c, j, p, y, k, e is obtained as a molecule-function network.

Based on the obtained "molecule-function network," it is possible to carry out presumption of bio-events as follows. When a biomolecule e is a key molecule and has information on a bio-event E, it is possible to presume that biomolecules c, j, p, y, k relate to the expression of the bio-event E directly or indirectly. Moreover, when there is information on up-or-down of a bio-event such that decrease of molecule e elevates the expression of bio-event E, it is possible to presume the effect of quantitative or qualitative changes of arbitrary molecules out of c, j, p, y, k to the expression of the bio-event E, considering relations of (c, j) (j, p) (p, y) (y, k) (k, e).

Furthermore, it is possible to predict the effect on the amount of bio-event expression $Q_E$ given by N biomolecules on a molecule-function network from a certain biomolecule to a key molecule, by the following formula, for example. Here, $S_i$ is a qualitative evaluation value of the condition of the i-th biomolecule, $R_i$ is a value representing the amount of the i-th biomolecule, $V_i$ is an evaluation value of the environment where the i-th biomolecule exists, and f is a multiple-valued function with 3 × N input values.

$$Q_E = f(S_1, R_1, V_1, \ldots S_N, R_N, V_N)$$

Whereas the kinds of bio-events relating to one biomolecule-function network is not limited to one and it is expected that there are several molecule-function networks related to one kind of bio-event, it is possible to screen related molecule-function networks from the side of bio-events. For example, if a "molecule-function network" containing enormous numbers of biomolecules is generated by designating one or more biomolecules, it is possible to screen the range of the "molecule-function network" by adding information on bio-events. As a matter of course, it is also possible to generate a "molecule-function network" provided that some kind of mediator molecule, or relation between said molecule and a target biomolecule is included.

Moreover, it is possible to generate a molecule-function network within a necessary range by dividing, filtering, extracting subset from, and/or hierarchizing the data of "biomolecule-linkage database" appropriately. Dividing, filtering, and extracting subset can be carried out by search methods such as a search to the data items specific to the database of the present invention, a general text search using keywords, a homology search to amino acid sequences or nucleic acid sequences, a substructure search to chemical structures. By carrying out these searches to the "biomolecule-linkage database" or the "biomolecule information database" beforehand, it is possible to generate a restricted molecule-function network or a characterized molecule-function network. For example, it is possible to generate a "molecule-function network" with restricted range by generating a partial database screened from viewpoints such as biomolecule generated in liver and bio-events occurring in skin using the information on originating organs or acting organs, and carrying out a connect search. Furthermore, it is possible to generate a molecule-function network with desirable characteristics or with desirable range by dividing, filtering, and/or extracting subset of the molecule-function network generated by a connect search, carrying out the aforementioned search to biomolecules or biomolecule pairs included therein. Such restriction and characterization not only facilitate the search, but also are effective for helping one to understand the molecule-function network by highlighting a specific group of biomolecules or biomolecule pairs on the molecule-function network.

By dividing, filtering and/or extracting subset of the "biomolecule-linkage database" appropriately based on the linkage on the network, and by storing and using information indicating its inclusive relation, it is possible to hierarchize the "molecule-function network." Even when there are some unknown molecules or unknown linkages between molecules, it is possible to generate a tentative molecule-function network by combining them to one virtual biomolecule respectively and defining a pair with other molecule. When an extremely complicated network is generated because of the enormous number of the molecules included therein, it is possible to describe the network simply by defining two or more biomolecules linked in the network as one virtual biomolecule respectively.

Use of such hierarchies makes it possible to speed up a connect search, and to avoid extreme complexity appropriately by making precision of the network description adjustable. In the present description, such a partial network consisting of two or more biomolecule pairs linked in the network is called a "subnet".

Any partial network can be designated as a subnet, however, preferably, it is convenient to treat cascade, pathway and/or cycle, which is well-known to researchers like TCA cycle and pentose phosphate cycle in the metabolic system, as a subnet. Furthermore, a certain subnet may be included in a different subnet, for example, the metabolic system itself may be regarded as an upper subnet including multiple subnets.

Although there is a method of treating each subnet as one virtual biomolecule, it is convenient to store information on biomolecule pairs constituting a subnet and information on the hierarchy of the subnet in the "biomolecule-linkage database". Moreover, one may set up an upper data hierarchy to represent a subnet in the "biomolecule-linage database" and store therein the information on said subnet. The hierarchization of biomolecule pairs by subnet is not limited to two layers, and one may store a group of multiple subnets as a still upper subnet. In order to facilitate cross-referencing between the molecule pair data and the upper-hierarchy subnet data at the time of the network generation, it is desirable to store information indicating mutual relation between molecule pair and subnet, respectively in the molecule pair data and in the subnet data. It is needless to say that one biomolecule pair may be related to multiple subnets.

It is desirable to include not only the links to biomolecule pairs in lower hierarchy but also the information on relation between subnets in the subnet data of the hierarchized "biomolecule-linkage database". For example, glycolytic pathway and TCA cycle are subnets working in order in the metabolic system, and it is possible to store the relation between these subnets as a pair in upper hierarchy. In this case, it is desirable to add information on biomolecules that become contact points between the subnets in addition to the information on the subnet pair.

Furthermore, besides hierarchization of networks, biomolecules themselves can be hierarchized, and its information can be stored and used in the "biomolecule information database," which is one of the characteristics of the present invention. For rapid search and convenient and various display of the network, it is desirable to hierarchize both information on biomolecules and on biomolecule pairs. Items to be hierarchized for biomolecules can be exemplified as follows. Among biomolecules, there are cases in which multiple different molecules gather specifically to express a certain function, and there are also many cases in which expressing state and kind of functions are controlled depending on the difference in complexation states of molecules. Furthermore, as observed in immunocytes, there are cases in which relations to bio-events or cell functions are determined by the combination of multiple molecules expressed on the cell surface. In such cases, there is a method of treating the complexation state of molecules as one virtual biomolecule as described above, but as another method, one may set up an upper data hierarchy to represent the complexation state of molecules in the "biomolecule information database" and store the information on said complexation state therein. In order to facilitate cross-referencing between the biomolecule data and the upper hierarchy data at the time of generating the molecule-function network, it is desirable to store information representing mutual relation between the biomolecule data and upper hierarchy data, respectively in the biomolecule data and in the upper hierarchy data. It is needless to say that one biomolecule may be related to multiple upper hierarchy data.

Among bio-events and pathological events, there are many that cannot be related to a specific biomolecule pair. For example, there are cases in which a relation between a bio-event or pathological event and formation of a certain subnet is known, but the biomolecule pair to which said event is directly related is unknown. In such cases, it becomes possible to describe the relation between said event and the biomolecule network by relating the bio-event or pathological event to the subnet data which is an upper hierarchy of the biomolecule pair, using the aforementioned hierarchization of biomolecule pair data.

Furthermore, when a complexation state of specific molecules or an expression state of certain molecules on cell surface is related to the expression of a certain bio-event or pathological event, it becomes possible to describe the relation between said event and the biomolecule network by relating the bio-event or pathological event to the complexation state of molecules or the expression state of molecules using the aforementioned hierarchization of complexation state of molecules or expression state of molecules.

Furthermore, among bio-events and pathological events, there are some that can be related neither to a specific biomolecule pair nor to a subnet. An example of such cases is a pathological event "inflammation" which is caused by combination of various bio-events such as the release of inflammatory cytokines, infiltration of leukocytes to tissue, and increase in permeability of capillary vessel. In order to handle such an event, it is preferable to hierarchize bio-events and pathological events, describe events that can be related to biomolecule pairs and subnets in the lower hierarchy, and describe event that occurs in relation with the events in the lower hierarchy in the upper hierarchy. It is needless to say that more than two levels of hierarchy may be used this hierarchization. In order to facilitate cross-referencing events between hierarchies, it is desirable to store information indicating relations to the data in the upper and lower hierarchies in event data in each hierarchy. By such hierarchization of data of bio-events and pathological events, it becomes possible to describe the relation with molecule-function networks for those events that cannot be related directly to a specific biomolecule pair or a subnet.

As exemplified above, by hierarchizing and storing the data in "biomolecule information database" and "biomolecule-linkage database," it becomes possible to carry out the generation of molecule-function networks effectively corresponding to various purposes.

When a relation between a certain biomolecules (molecule A) in the glycolytic pathway and a certain protein (molecule B) in a certain kinase cascade is examined, it is necessary to carry out a connect search with enormous number of molecule pairs if we use data without hierarchization, and the search is practically impossible when the path between molecule A and molecule B is too long. On the other hand, using the hierarchized data, it is possible to carry out a connect search between the subnet "glycolytic pathway" and the subnet "certain kinase cascade" in the upper hierarchy, namely subnets, and if path is found in the upper hierarchy, it is possible to carry out a connect search in the lower hierarchy of each subnet on that path as necessary. Thus, by dividing a pathway search problem to the problems in different hierarchies, it becomes possible to generate a molecule-function network that was impossible without hierarchization.

Furthermore, when a specific subnet is frequently referred to in a connect search using the aforementioned hierarchized data, it is recommended to carry out a connect search beforehand within said subnet, and store the information on the molecule-function network in said subnet. With this process, it becomes possible to generate the entire molecule-function network more effectively.

Furthermore, when a molecule-function network related to the pathological event "inflammation" is generated, for example, it becomes possible to generate a more extensive molecule-function network by searching events in lower hierarchy related to the event "inflammation" of upper hierarchy, and by carrying out connect searches starting from biomolecule pairs or subnets to which said events of lower hierarchy are related.

As described above, by the present invention, it is possible to generate molecule-function networks relating to arbitrary molecules based on the information on relations of direct-binding biomolecules, and to presume easily the bio-events and pathological events that are related directly or indirectly. Furthermore, the present invention can be used inversely for the purpose of selecting a molecule-function network with high possibility of relation with a disease based on the characteristic findings in the disease such as bio-events, pathological events and changes in the amounts of biomolecules, and predicting molecular mechanism of the disease. Moreover, by the present invention, it becomes possible to construct strategies for drug development such that inhibition of which process in the network is effective for treatment of a specific disease or a symptom, which molecule in the network is promising as a drug target (a protein or other biomolecule to be targeted in drug development), what kind of side effects are expected from the drug target, and what kind of assay system is appropriate for selecting drug candidates while avoiding the side effects.

A drug molecule, in general, exerts its pharmacological activity by binding to a biopolymer such as a protein in an organism and by controlling its function. The actions of those molecules have been studied more precisely compared to the actions of biomolecules, contributing to the elucidations of molecular mechanisms of target diseases. Thus, we noticed that the usefulness of the methods of the present invention is enhanced by adding relations of pairs between a drug molecule approved for manufacturing and used for medical treatment or a drug molecule used for pharmacological studies and its target biomolecule, to the aforementioned information on biomolecules and biomolecule pairs. In most cases, target biomolecules are proteins or proteins modified with sugars. It becomes possible to presume bio-events that are likely to be side effects based on the molecule-function network including the target biomolecule, and it also becomes possible to presume interaction between drugs from crossovers in the molecule-function networks relating to drugs administered together. As a result, it becomes possible to select and determine dose of a drug while considering risk of side effects and risk of interaction between drugs.

Examples of the methods of the present invention wherein relations between a drug molecule and a target biomolecule are added are described below. A molecule ID is defined for the formal nomenclature of each drug molecule, and a "drug molecule information database" is prepared which stores all information on said molecule itself. For each drug molecule, the name, molecule ID, indications, dose, target biomolecules and other information are stored herein. As in the case of the biomolecule information database, information such as the chemical structure, amino acid sequence (in case of peptides or proteins) and steric structure of drug molecules may be included in the "drug molecule information database", but it is preferable to store them in a separate database. For the purpose of discriminating between drug molecules and biomolecules or between proteins and small molecules, one may use discrimination by structure code and others, or employ a rule of assigning molecule IDs wherein the first letter tells the difference, for example. Furthermore, if information such as the remarkable side effects, interaction with other drugs, and metabolizing enzymes are input from prescribing information or other literature about drugs, it will be helpful for the purpose of appropriate selection of a drug in relation to gene polymorphism based on the molecule-function network.

Furthermore, a "drug molecule-linkage database" which is a database containing the information on pairs of a drug molecule and a target protein as well as the information on their relation may be prepared. Molecule ID of drug molecule, molecule ID of target biomolecule, relation code, pharmacological action, indication and other information regarding the drug molecules are stored therein. Concerning the molecule IDs of the target biomolecules, it is necessary to use those defined in the biomolecule information database. Concerning data items common to the biomolecule-linkage database such as relation code, it is preferable to use description rules conforming to those of the biomolecule-linkage database.

By preparing the "drug molecule information database" and "drug molecule-linkage database" and importing information on drug molecules and drug molecule pairs therein, the method of the present invention can be expanded as shown in Fig. 2. Here, the generation of a molecule-function network and presumption of bio-events by a connect search can be carried out by a method similar to the aforementioned method wherein only biomolecule-linkage database and biomolecule information database are used, and information on known drug molecules that target molecules on said network is obtained as well. Furthermore, it is useful for the purpose of extracting a molecule-function network to which a designated drug molecule is related from the molecule-function networks that has been generated using only the biomolecule-linkage database and biomolecule information database.

On the other hand, elucidations of genetic information from various aspects are progressing rapidly including the analysis of human genome sequence. cDNAs are isolated in genome-wide scale, elucidations of orf (open reading frame) and gene sequences are progressing, and locating of genes on the genome is proceeding. Hereupon, as further embodiments of the present invention, the present invention can be expanded as follows by preparing a biomolecule-gene database which relates molecule IDs of proteins among biomolecules to the information of the genes coding said proteins comprising their names, abbreviated names, IDs and others. That is, correlating genes and biomolecules makes it possible to understand the meaning of genes and proteins which are the markers of a disease and the findings such as a relation between a disease and a gene polymorphism, in relation with molecules and bio-events in the molecule-function network. In the biomolecule-gene database, it is preferable to include information such as the amino acid mutation and abbreviation of gene polymorphism, and relation with functions as well as the species, location on the genome, gene sequence and function, and it is acceptable to prepare two or more databases if necessary.

Based on the gene names located on genome sequences or the arrangement of genes, proteins that are translated by the action of a specific key molecule to a nuclear receptor are identified, making it possible for relations of mutual control between biomolecules to be reflected on the molecule-function network. Furthermore, it is known that expressions of genes and proteins are different depending on organs, and by the method of the present invention, importing such expression information into the "biomolecule information database" makes it possible to generate different "molecule-function network" for each organ, and it becomes possible, for example, to explain a phenomenon such that a drug molecule targeting a nuclear receptor exerts different or inverted actions in different organs. Moreover, as it is known that expressions of proteins change upon administration of a drug molecule, interpreting the increase or decrease of amount of protein expression on the molecule-function network related to the target protein by the method of the present invention is useful for choosing drugs under consideration of the gene polymorphism.

Also in the aforementioned storage of information on gene transcription and protein expression, use of the concept of hierarchization makes it possible to generate molecule-function networks more effectively and broadly. For example, for multiple genes and/or proteins that are transcribed or expressed by a specific nuclear receptor, it is preferable to set up upper hierarchy representing the transcription of gene group and/or expression of protein group in the "biomolecule information database" and to store the data of said gene group and/or protein group therein. When there are bio-events and/or pathological events related to the transcription of said gene group and/or expression of said protein group, describing relations between upper hierarchy data of said gene group and/or said protein group and said event in the "biomolecule-linkage database" makes it possible to generate molecule-function networks that cannot be described with the relation between individual gene or molecule and said event.

In the aforementioned method of hierarchical storage of information on gene transcription and protein expression, if quantitative information on transcription or expression of individual gene of said gene group or individual protein of said protein group is available, it is preferable to store that information as numerical parameters in the "biomolecule information database". Using these numerical parameters, it becomes possible to describe the cases in which relating bio-events and/or pathological events change depending on the differences of the amount of expression of individual gene or the amount of expression of individual protein.

Furthermore, the diversity among individuals regarding a genome and genes has been made clear, and linking such information to the methods of the present invention makes it possible to progress understanding about the diversity among individuals and enables medical treatment based on the diversity. For gene polymorphism such that a function of a specific biomolecule (protein) is impaired, interpreting it on the molecule-function network makes it possible to presume its influence on bio-events. It is advantageous for understanding to link information on symptoms and abnormalities of bio-events in a genetic disease caused by a defect or an abnormality of a single gene to the methods of the present invention.

In several typical diseases, several genes frequently observed in patients with the disease, namely disease-related genes, have been reported to exist. Supposing genetic habitus prone to suffer from a specific disease actually exists, there can be two or more molecule-function networks related to, for example, the adjustment of blood pressure, and it is no wonder that considerable number of genes that might be related to the high blood pressure depending on the abnormality of any one of the molecules in any one of the networks. In order to interpret such a problem of polygenic genes, the methods of the present invention are indispensable.

Moreover, analyses of genomes and genes of animals such as mouse and rat have been progressing rapidly in recent years, and it is now possible to correspond those to human genome and genes. It is expected that proteins related to the regulation of physiological functions are considerably similar between these animals and human, however, the existence of appreciable differences has been an obstacle in drug developments. More cases are emerging in which proteins and protein functions are quite different between these animals and human, and it is useful for drug discovery to clarify the difference from the molecule-function network in human by linking them with the methods of the present invention. Moreover, for animal drugs that have been switched in many cases from drugs originally developed for human, these methods are also useful for aiming at their appropriate use.

In drug developments, when there is a disease model animal having similar pathological findings to a human disease, the development is carried out with the pharmacological activities in that animal as indices, in many cases. Studies on genes of such disease model animals are also progressing, and relating them to the genetic information of human by the methods of the present invention will be helpful for elucidating a mechanism of said human disease.

Furthermore, for the purpose of elucidating a gene function, there are more and more cases where one creates a knockout animal in which a specific gene is disabled or a transgenic animal in which a gene is changed to the gene with weaker function or to the over expressing gene. There are many cases where these are lethal and unable to be born or no influences are found in the biological functions or behaviors, and even in cases where a certain abnormality is found in a newborn animal, it is believed to be very difficult to analyze the result of these animal experiments. In such experiments, it is convenient to carry out functional analyses after predicting influences of said gene operation using the methods of the present invention.

Attempts of integrating information related to genes from aspects of sequence IDs are progressing, along with the progress of genome analysis, and furthermore, attempts of locating genes on the genome sequence are also progressing. It is possible to construct an original genetic information database considering cooperation with the aforementioned "biomolecule-linkagen database" and use it for the aforementioned purpose, however, taking into account the fact that those information are enormous and tend to be open to public, it is highly possible that the aforementioned methods can be carried out by incorporating such public information into the methods of the present information pro re nata in the future (Fig. 3).

Biomolecule-linkage databases used in the methods of the present invention are not necessarily managed and/or stored at the same site, and by unifying molecule IDs, one may select appropriately one or more biomolecule-linkage databases managed and/or stored at different sites and use them by connecting with communication means and others. It is needless to say that similar disposition is possible not only for the biomolecule-linkage database, but also for the biomolecule information database, drug molecule-linkage database, drug molecule information database, and gene information database used in the methods of the present invention.

As a still further embodiment of the present invention, there is also provided a method of preparing a database comprising information on biomolecules directly related to the expression of bio-events and said bio-events (a bio-event-biomolecule database) and using it with molecule-network databases that do not necessarily contain information on bio-events. As a still further embodiment, there is also provided a method of extracting partial molecule networks related to arbitrary molecules from molecule-network databases that do not necessarily contain information on bio-events, and searching the aforementioned bio-event-biomolecule database based on the molecules constituting said networks.

As a still further embodiment of the present invention, there is provided a method of searching based on keyword and/or numerical parameter and/or molecular structure and/or amino acid sequence and/or base sequence and others through data items in "biomolecule information database", "biomolecule linkage database", "drug molecule information database", "drug molecule-linkage database", "biomolecule-gene database" and others, and generating a molecule-function network based on the result of said searching. Examples of generating a molecule-function network based on the search are described below, however, it is needless to say that the scope of the present invention is not limited to these examples.

In each database, various information such as molecule names, molecule IDs, species, originating organs and existing organs are stored as texts. By searching through these texts based on the complete match or partial match of character strings, it is possible to screen biomolecules, biomolecule pairs, bio-events, pathological events, drug molecules, drug molecule-biomolecule pairs, gene-protein correspondence data and others. Based on these screened information, it is possible to define one or more starting point and/or end point of a connect search or limit molecule pairs used in the connect search, making it possible to generate molecule-function networks appropriate for its usage.

When chemical structures and/or steric structures of drug molecules are stored in the "drug molecule information database", carrying out a search based on full-structure match or sub-structure match or structure similarity makes it possible to screen drug molecules. Based on the screened drug molecules, it becomes possible to generate molecule-function networks related to said drug molecules and search bio-events and/or pathological events related to said drug molecules.

When numerical parameters such as those of gene transcription and protein expression are stored in the "biomolecule information database," carrying out a search based on these numerical parameters makes it possible to generate molecule-function networks corresponding the amounts of gene transcription and/or protein expression.

When amino acid sequences of proteins are stored in the "biomolecule information database" or in a related database, carrying out a search based on sequence homology or match of partial sequence pattern to these amino acid sequences makes it possible to screen biomolecules and generate molecule-function networks based on said biomolecules. This method is effective, concerning a protein with unknown function or its partial sequence information, for predicting molecule-function networks with which said protein fairly possibly has relations and for further predicting functions of said protein.

When base sequences of genes corresponding to proteins are stored in the "biomolecule information database", "biomolecule-gene database" or a related database, carrying out a search based on sequence homology or match of partial sequence pattern to these base sequences makes it possible to screen biomolecules and generate molecule-function networsk based on said biomolecules. This method is effective, concerning a gene with unknown function or its partial sequence information, for predicting molecule-function networks with which a protein translated from said gene fairly possibly has relations and for further predicting functions of said protein.

As still further embodiments of the present invention, there are provided a computer system consisting of programs and databases to carry out the methods of the present invention; a computer-readable medium storing programs and databases to carry out the methods of the present invention; a computer-readable medium storing databases to be used by the methods of the present invention; a computer-readable medium storing information on the molecule-function networks generated by the methods of the present invention..

Characteristics of the methods of the present invention are as follows.

- By accumulating information on direct-binding biomolecule pairs having information on bio-events, a database of relations between molecules in an organism is generated.
- By a connect search to the aforementioned database which is a collection of parts, a molecule-function network related to one or more arbitrary biomolecules or bio-events is generated.
- Based on the molecule-function network, bio-events to which one or more arbitrary molecule is directly related are presumed.
- From the molecule-function network with information on one ore more bio-events, a mechanism of a disease, a possible drug target, a risk of a side effect and others are presumed.
- From quantitative or qualitative changes of biomolecules, up-or-down of one ore more bio-events are presumed.
- A molecule-function network having information on originating organs, existing organs and acting organs of biomolecules.
- Presumption of side effects and interactions between drugs using the drug molecule information and the molecule-function network.
- Interpretation of changes of protein expression upon administration of a drug molecule on the molecule-function network.
- Analyses of influences of gene polymorphism on the molecule-function network, disease-related gene and others by linking to genetic information.

## Examples

In the following, the present invention is explained with examples more specifically, however, the scope of the present invention is not limited to these.

### Example 1

An example of generating molecule-function networks for rennin-angiotensin system is shown. Rennin-angiotensin system is one of the main mechanisms of adjustment of blood pressure in an organism, and many of the related biomolecules have been revealed (Fig.4). For biomolecules related to the rennin-angiotensin system known so far, a biomolecule information database (Fig.5) and a biomolecule-linkage database (Fig. 6) were generated, and generations of molecule-function networks were tried by giving biomolecules and bio-events as queries.

Fig.7 shows a molecule-function network that was generated by giving "angiotensin I" which is one of the biomolecules and "blood pressure increase" which is one of the bio-events as queries. By carrying out a connect search to the biomolecule-linkage database, biomolecules related to "angiotensin I" through "blood pressure increase" and a molecule-function network generated thereby were obtained.

Furthermore, a drug molecule information database (Fig.8) and a drug molecule-linkage database (Fig.9) were generated for drug molecules having a hypotensive action, and a trial of generating a molecule-function network to which a drug molecule is related was carried out by using these databases together with the biomolecule information database (Fig.5) and the biomolecule-linkage database (Fig.6).

In Fig.10, a molecule-function network generated by giving "enalapril" which is one of the drug molecules and "blood pressure increase" which is one of the bio-events as queries is shown. Since enalapril has a relation of inhibition to direct-binding angiotensin-converting enzyme, a link to angiotensin II having a direct-binding relation (enzyme-substrate relation) to angiotensin-converting enzyme is broken, and it is shown that an event of "blood pressure increase" existing on the subsequent network is suppressed (stopped).

### Example 2

An example of implementation of the present invention as a program for searching and displaying molecule-function networks is shown. Fig.11 shows a flow chart of the searching and displaying of the present example, but these processes only indicate an example of implementation of the present invention as a program, and it is needless to say that the scope of the present invention is not limited to this example.

This program comprises steps from 1101 to 1103 wherein a search is carried out to obtain molecule names, subnet names, or bio-event names necessary for carrying out a connect search, steps from 1104 to 1108 wherein a connect search is carried out and a molecule-function network is displayed, and additional steps from 1109 and 1110 wherein the generated molecule-function network is further processed.

First, a user designates the search method for molecule name, molecule ID, subnet name, bio-event name, pathological event name, disease name, amino acid sequence, nucleic acid sequence, external database ID, drug molecule structure and others in step 1101, and inputs a query character string. As for the search method, the user can choose among a method of carrying out a search individually to the aforementioned items, a method of carrying out a search with a common query character string to multiple items, and others. The query character string is not necessarily the one exactly matching the data item in the database, but the one representing some part of the name or the one containing so-called wild-card characters is acceptable. When an amino acid sequence of a protein or a nucleic acid sequence is designated as a query item, the user inputs a character string representing the amino acid sequence or the base sequence with 1 letter code (for example: alanine=A, glycine=G, guanine=g, cytosine=c and the like) as the query character string. When a drug molecule structure is designated as a query item, the user inputs data representing the query molecular structure in the format of MOLFILE and others.

For the search items which the user input, the program carries out a search in step 1102 to the data items of the biomolecule information database, biomolecule-linkage database and related databases, by methods of keyword search, molecular structure search, sequence search and others. In the keyword search, not only a full match of the character string, but also a partial match of the character string or a match to the multiple character strings by wild-cards may be acceptable. When an amino acid sequence or a base sequence is designated as a query item in step 1101, the program carries out a search by identity or homology of the query character string (sequence) to amino acid sequences or base sequences in the biomolecule information database or related sequence databases, and returns IDs or corresponding molecule names of sequences with high degrees of identity or homology as a search result. When a drug molecule structure is designated as a query item, the program searches drug molecules whose partial structures are identical or similar by the method of substructure matching, and returns corresponding drug molecule names as a search result.

Hit items obtained by the search in step 1102 are displayed as a list in step 1103. The program displays hit items on the list distinctively whether they are molecule names, subnet names or bio-event names, by separating locations in the list or by adding icons.

Next, the user designates the method of connect search and molecule names, subnet names or bio-event names (including pathological events) which will be the endpoints in step 1104. In this example, a method of searching a network connected around one designated point and a method of searching a network connecting two designated points are provided as the methods of connect search. Input items necessary for these two kinds of search methods are shown in Fig.12 and Fig. 13, respectively. The user inputs one or more molecule names, subnet names or bio-event names by selecting appropriate items from the list displayed in step 1103. When there is no appropriate item on said list, the user can return to the input of query items in step 1101 and can repeat the search process of step 1101 through step 1103 until an appropriate item is found.

In step 1105, the user inputs one or more restricting conditions for the connect search. As the restricting conditions, the user can designate an upper limit to the number of molecules included in the molecule-function network to be generated, an upper limit to the number of relations (number of paths) intervening said two points when searching between two endpoints, and others. In step 1106, the user designates the method of displaying the molecule-function network obtained as a result of the search. As the displaying method, the user can choose among a method of displaying all molecules constituting the network explicitly (molecule-network display), a method of displaying molecules belonging to a subnet bundled as one node (subnet display), and others.

According to the designated conditions in step 1104 to step 1105, the program carries out a connect search to the biomolecule-linkage database in step 1107. The molecule-function network obtained as a result of the search is displayed as a graph having molecules, subnets, or bio-events as nodes in step 1108, according to the displaying method designated by the user in step 1106.

The user examines visually the molecule-function network displayed in step 1108, can go back to step 1104 to change the conditions of connect search and repeat searches as necessary, and can go back to step 1101 to repeat the search of molecule names, subnet names, or bio-event names.

Furthermore, the generated molecule-function network can be further processed with an additional step 1109 or 1110 in this program. In step 1109, the user can carry out logical operations between multiple molecule-function networks. For carrying out step 1109, it is necessary to generate multiple molecule-function networks by carrying out the processes to step 1108 multiple number of times. For these multiple molecule-function networks, the program can derive a common part (AND operation) or non-common parts (XOR operation) between networks, and can derive a logical sum (OR operation) of multiple networks. This function is useful for examining differences of molecule-function networks in different species, organs and others.

In step 1110, the user can further carry out a screening search to the generated molecule-function network, and can highlight or extract molecules or partial networks in said molecule-function network. In this screening search, any search method used in steps 1101～1103 can be used. With step 1110, it becomes possible, for example, to highlight biomolecules expressed in a specific organ in the molecule-function network, and to extract and display only those parts belonging to designated subnets in a broad molecule-function network.

Industrial Applicability

The biomolecule-linkage database of the present invention which is a collection of information on biomolecule pairs including bio-events is useful for generating a molecule-function network with a necessary range which is a functional or biosynthetic linkage between molecules and predicting bio-events to which an arbitrary biomolecule is related directly or indirectly, and furthermore, by linking it to the information on drug molecules or genetic information, it is possible to obtain necessary knowledge for drug developments and medical treatments based on differences of individuals.

## Claims

1. A method of generating a molecule-function network containing bio-event(s) by carrying out a connect search using a biomolecule-linkage database containing information on the bio-event(s).

2. A method of generating a molecule-function network containing bio-event(s) by carrying out a connect search using a biomolecule-linkage database containing information on the bio-event(s) and predicting a pathway between an arbitrary biomolecule and an arbitrary bio-event in said network.

3. A method of generating a molecule-function network containing bio-event(s) by carrying out a connect search using a biomolecule-linkage database containing information on the bio-event(s) and predicting the bio-event(s) with which an arbitrary biomolecule in said network is related.

4. A method of generating a molecule-function network by a connect search using a biomolecule-linkage database wherein an information on a biomolecule pair is hierarchically stored.

5. The method according to any one of claims 1 through 3, **characterized in that** an information on a biomolecule pair is hierarchically stored.

6. The method according to any one of claims 1 though 5, **characterized by** using a database wherein an information on the biomolecule and/or the bio-event(s) is hierarchically stored.

7. The method according to any one of claims 1 through 6, **characterized by** carrying out any one of, or two or more of keyword search, molecular structure search, or sequence homology search to items in the database.

8. The method according to any one of claims 1 through 6, **characterized by** screening a datum used for a connect search by carrying out any one of, or two or more of keyword search, molecular structure search, or sequence homology search to items in the database, and generating a limited molecule-function network.

9. The method according to any one of claims 1 through 6, wherein any one of, or two or more of keyword search, molecular structure search, or sequence homology search are further carried out to the generated molecule-function network for generation of a partial network of said network.

10. The method according to any one of claims 1 through 6, **characterized in that** the information on the bio-event(s) includes information of up-or-down corresponding to a quantitative or qualitative change of a key molecule.

11. The method according to any one of claims 1 through 6, **characterized in that** the information on the bio-event(s) includes one or more kinds of information comprising a disease name, a disease state, a diagnostic criterion, and a therapeutic agent.

12. The method according to any one of claims 1 through 6, **characterized by** further using information on a drug molecule wherein a target of said drug molecule is a specific biomolecule.

13. The method according to any one of claims 1 through 6, **characterized by** further using information on a correspondence between a biomolecule and a gene.

14. The method according to any one of claims 1 through 6, **characterized by** further using information on a protein expression and/or a gene expression in each organ.

15. The method according to any one of claims 1 through 6, **characterized in that** the biomolecule is linked to information of a gene involved in a gene polymorphism.

16. The method according to any one of claims 1 through 6, **characterized by** further using information on a gene or a protein whose expression is regulated by a specific key molecule.

17. A method of predicting a side effect of a drug molecule, **characterized by** using the method of claim 12.

18. A method of predicting a drug target, **characterized by** using the method according to any one of claims 1 through 16.

19. A method of predicting a risk of a side effect when a specific biomolecule is selected as a drug target, **characterized by** using the method according to any one of claims 1 through 16.

**20.** A computer system comprising a program and a database for carrying out the method according to any one of claims 1 through 19.

**21.** A computer-readable medium storing a program and/or a database for carrying out the method according to any one of claims 1 through 19.

Fig.1

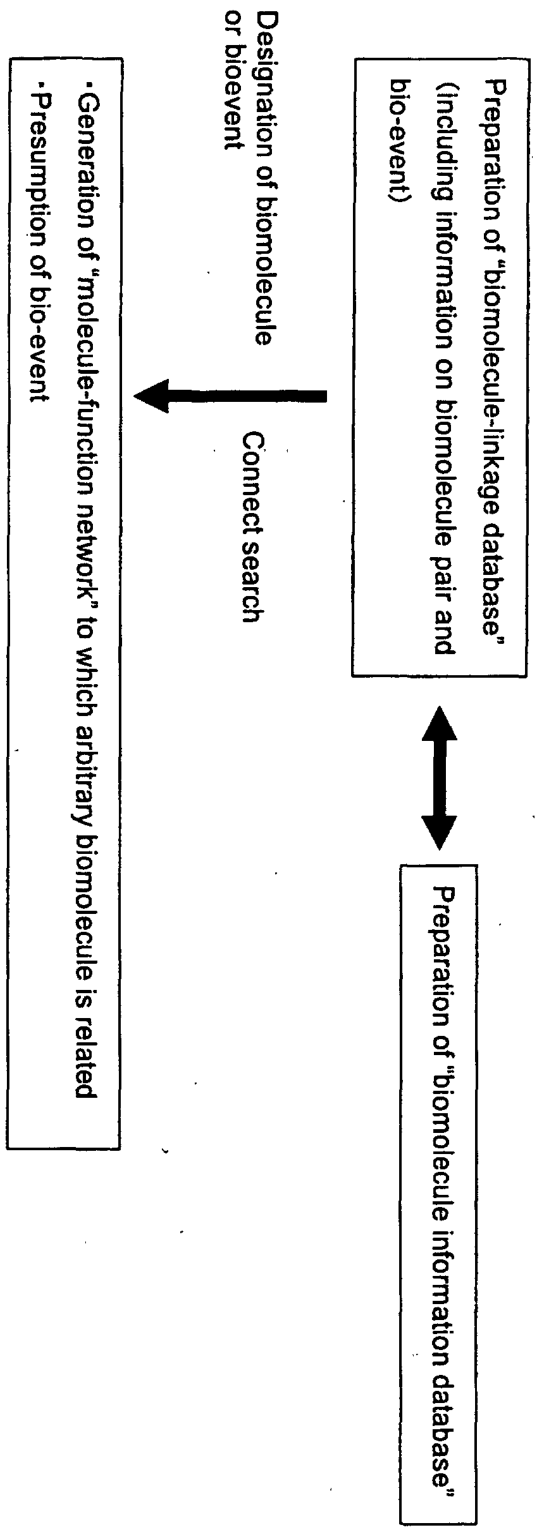
Preparation of "biomolecule-linkage database" (including information on biomolecule pair and bio-event)
Preparation of "biomolecule information database"
Designation of biomolecule or bioevent
Connect search
·Generation of "molecule-function network" to which arbitrary biomolecule is related
·Presumption of bio-event

Fig.2

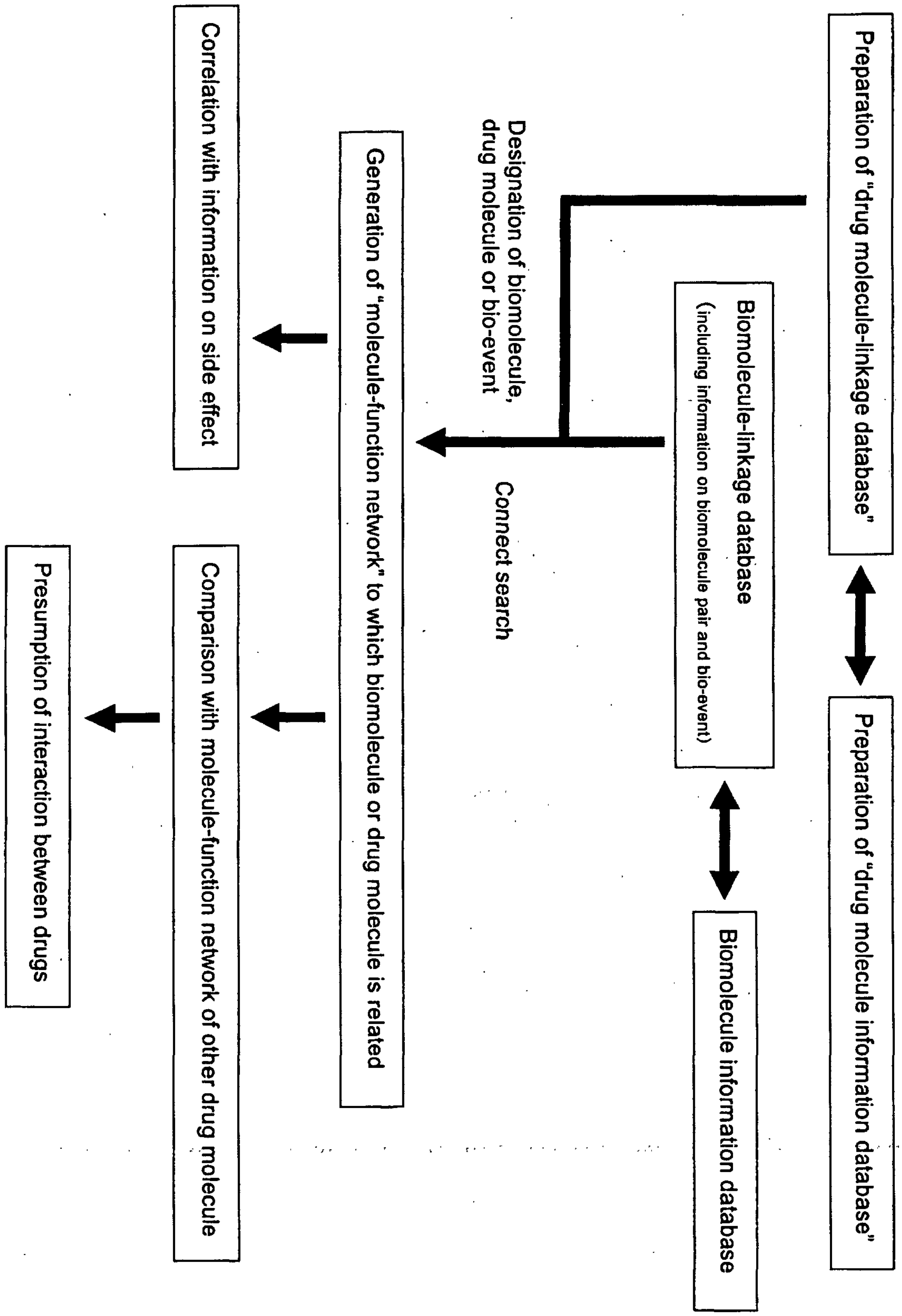
Preparation of "drug molecule-linkage database"
Preparation of "drug molecule information database"
Biomolecule-linkage database
(including information on biomolecule pair and bio-event)
Biomolecule information database
Designation of biomolecule, drug molecule or bio-event
Connect search
Generation of "molecule-function network" to which biomolecule or drug molecule is related
Correlation with information on side effect
Comparison with molecule-function network of other drug molecule
Presumption of interaction between drugs

Fig. 3

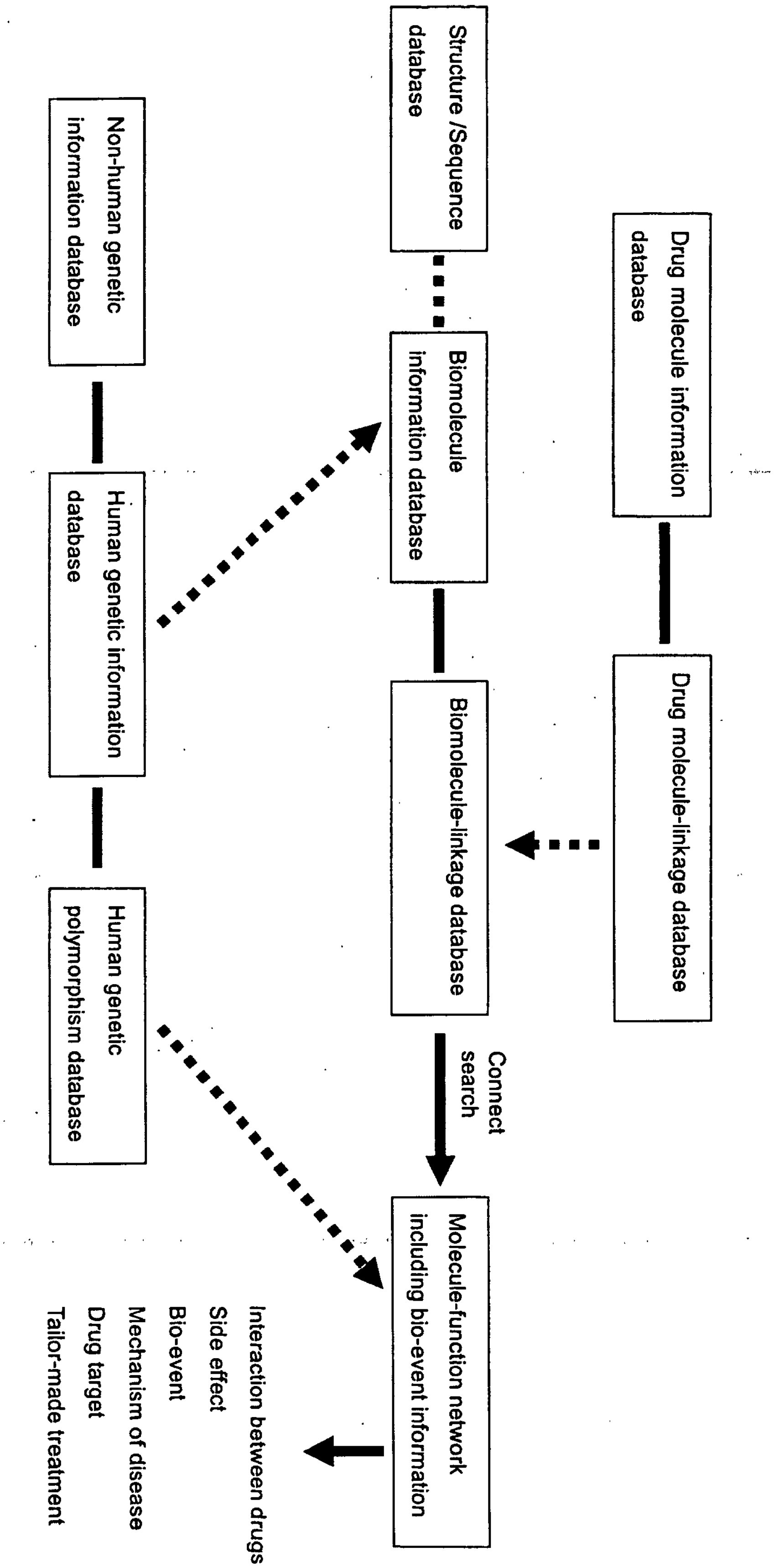
Drug molecule information database
Drug molecule-linkage database
Structure /Sequence database
Biomolecule information database
Biomolecule-linkage database
Connect search
Molecule-function network including bio-event information
Non-human genetic information database
Human genetic information database
Human genetic polymorphism database
Interaction between drugs
Side effect
Bio-event
Mechanism of disease
Drug target
Tailor-made treatment

Fig. 4

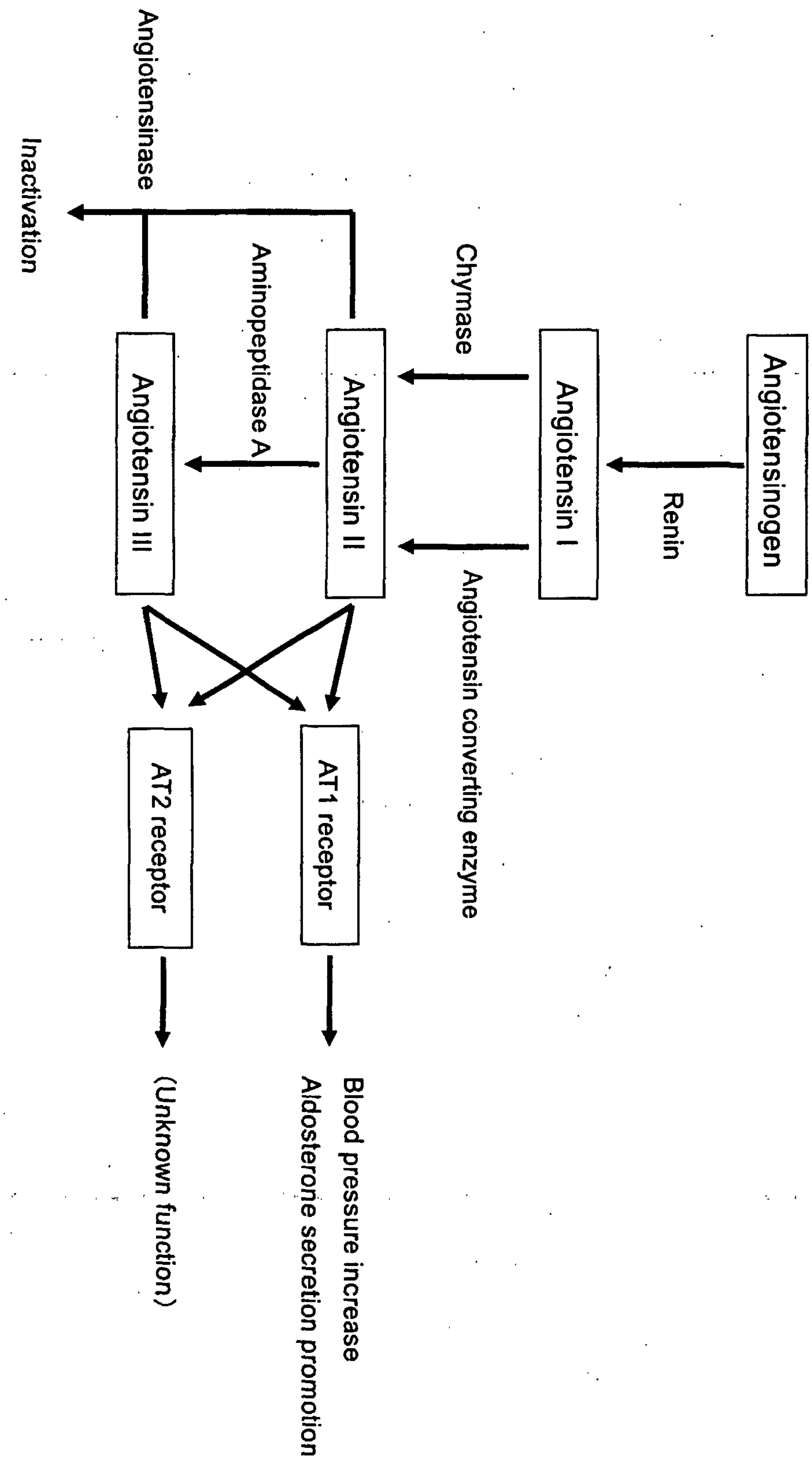
Angiotensinogen
Renin
Angiotensin I
Chymase
Angiotensin converting enzyme
Angiotensin II
Aminopeptidase A
Angiotensin III
Angiotensinase
Inactivation
AT1 receptor
AT2 receptor
Blood pressure increase
Aldosterone secretion promotion
(Unknown function)

Fig. 5

| Molecule name | Symbol | Molecule Code | Function Code | SwissProt Code | Originating Organ | Existing Organ |
|---|---|---|---|---|---|---|
| Renin | REN | Protein | Enzyme | RENI_HUMAN | Kidney (juxtaglomerular cell) | In blood |
| Angiotensin converting enzyme | ACE | Protein | Enzyme | ACE_HUMAN | Vascular endotherium | Vascular endothelium, In blood |
| Angiotensinase | PRCP | Protein | Enzyme | PCP_HUMAN | (Lysosome) | (Lysosome) |
| Chymase | CMA1 | Protein | Enzyme | MCT1_HUMAN | Mast cell, Heart | In blood |
| Aminopeptidase A | APA | Protein | Enzyme | AMPE_HUMAN | Kidney and others | Kidney and others |
| Angiotensinogen | ANGTS0 | Peptide | Precursor | ANGT_HUMAN | Liver | In blood |
| Angiotensin I | ANGTS1 | Peptide | Precursor | — | In blood | In blood |
| Angiotensin II | ANGTS2 | Peptide | Active peptide | — | In blood | In blood |
| Angiotensin III | ANGTS3 | Peptide | Active peptide | — | In blood | In blood |
| Angiotensin II metabolite | ANGTX2 | Peptide | Metabolite | — | In blood | — |
| Angiotensin III metabolite | ANGTX3 | Peptide | Metabolite | — | In blood | — |
| Angiotensin receptor I | AGTR1 | Protein | Membrane receptor | AG2R_HUMAN | Blood vessel、Adrenal gland | Blood vessel, Adrenal gland |
| Angiotensin receptor II | AGTR2 | Protein | Membrane receptor | AG22_HUMAN | Brain, Adrenal gland | Brain, Adrenal gland |

Fig. 6

| Molecule1 | Molecule2 | Relation Code | Acting Organ | Bio-Event | Pathological Event |
|---|---|---|---|---|---|
| ANGTNS0 | REN | 21 | In blood | — | — |
| REN | ANGTNS1 | 22 | In blood | — | — |
| ANGTNS1 | ACE | 21 | In blood (Lung circulation) | — | — |
| ACE | ANGTNS2 | 22 | In blood (Lung circulation) | — | — |
| ANGTNS1 | CMA1 | 21 | In blood | — | — |
| CMA1 | ANGTNS2 | 22 | In blood | — | — |
| ANGTNS2 | APA | 21 | In blood | — | — |
| APA | ANGTNS3 | 22 | In blood | — | — |
| ANGTNS2 | PRCP | 21 | In blood | — | — |
| PRCP | ANGTX2 | 22 | In blood | Blood Pressure Drop | — |
| ANGTNS3 | PRCP | 21 | In blood | — | — |
| PRCP | ANGTX3 | 22 | In blood | Blood Pressure Drop | — |
| ANGTNS2 | AGTR1 | 11 | Blood vessel, Adrenal gland | Vasoconstriction、Blood pressure increase | Hypertension |
| ANGTNS2 | AGTR2 | 11 | Brain, Adrenal gland | (Unknown) | (Unknown) |

Relation Code 11:Agonist—Receptor、 12:Antagonist—Receptor
21:Substrate—Enzyme、 22:Enzyme—Product、 23:Inhibitor—Enzyme、 50:Metabolite

Fig.7

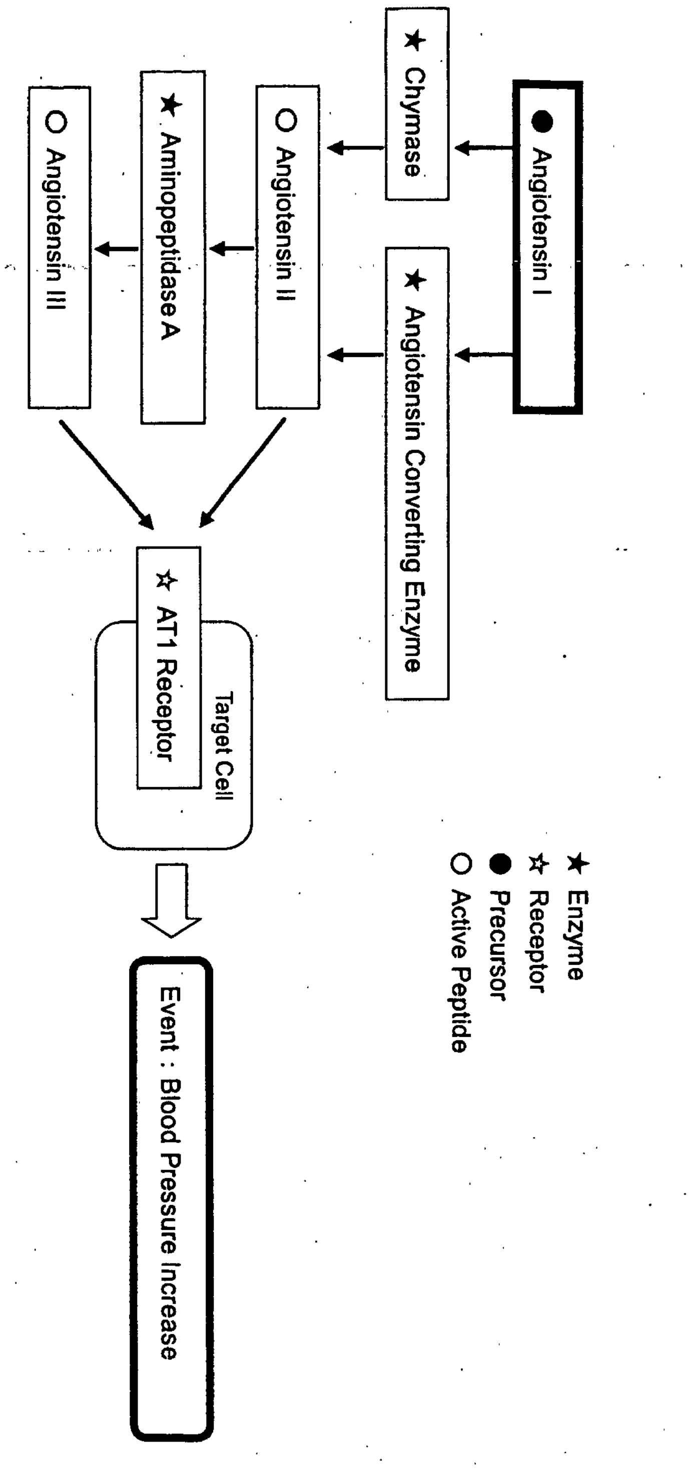
● Angiotensin I
★ Chymase
★ Angiotensin Converting Enzyme
○ Angiotensin II
★ Aminopeptidase A
○ Angiotensin III
☆ AT1 Receptor
Target Cell
Event : Blood Pressure Increase
★ Enzyme
☆ Receptor
● Precursor
○ Active Peptide

Fig.8

| Molecule Name | Abbreviation | Action | Target Disease | Side Effect |
|---|---|---|---|---|
| Enalapril | ENALAPR | Blood Pressure Drop | Hypertension, Chronic Heart Failure | Dry Cough, Acute Renal Failure, Angioedema |
| Losartan | ROSARTN | Blood Pressure Drop | Hypertension | Angioedema, Acute Hepatitis, Renal Failure |

Fig.9

| Molecule 1 | Molecule 2 | Relation Code | Acting Organ | Pharmacological Action |
|---|---|---|---|---|
| ENALAPR | ACE | 2 3 | In blood | Blood Pressure Decrease |
| ROSARTN | AGTR1 | 1 2 | Blood Vessel, Adrenal | Blood Pressure Decrease |

Legend of Relation Code 1 1 : Agonist—Receptor, 1 2 : Antagonist—Receptor
2 1 : Substrate—Enzyme, 2 2 : Enzyme—Product, 2 3 : Inhibitor—Enzyme, 5 0 : Metabolite

Fig.10

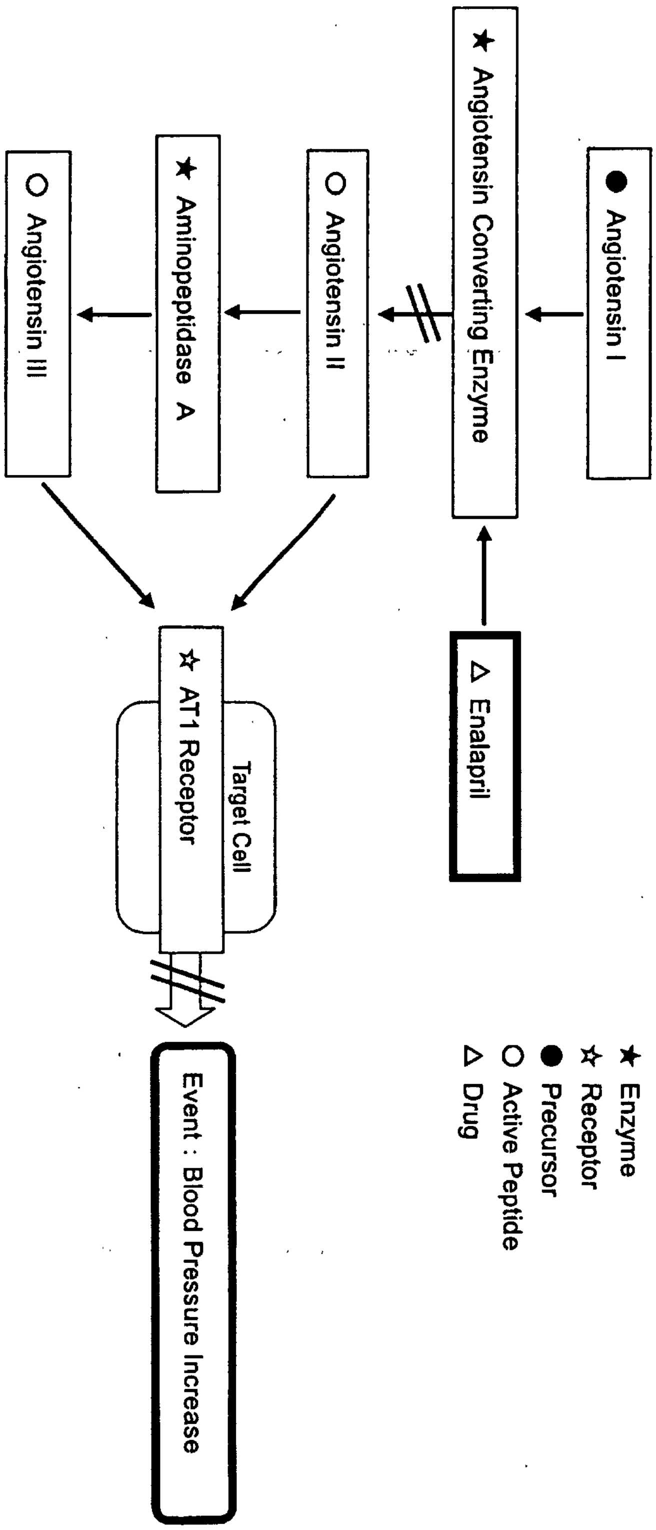
Angiotensin I
Angiotensin Converting Enzyme
Enalapril
Angiotensin II
Aminopeptidase A
Angiotensin III
AT1 Receptor
Target Cell
Event : Blood Pressure Increase
Enzyme
Receptor
Precursor
Active Peptide
Drug

Fig.11

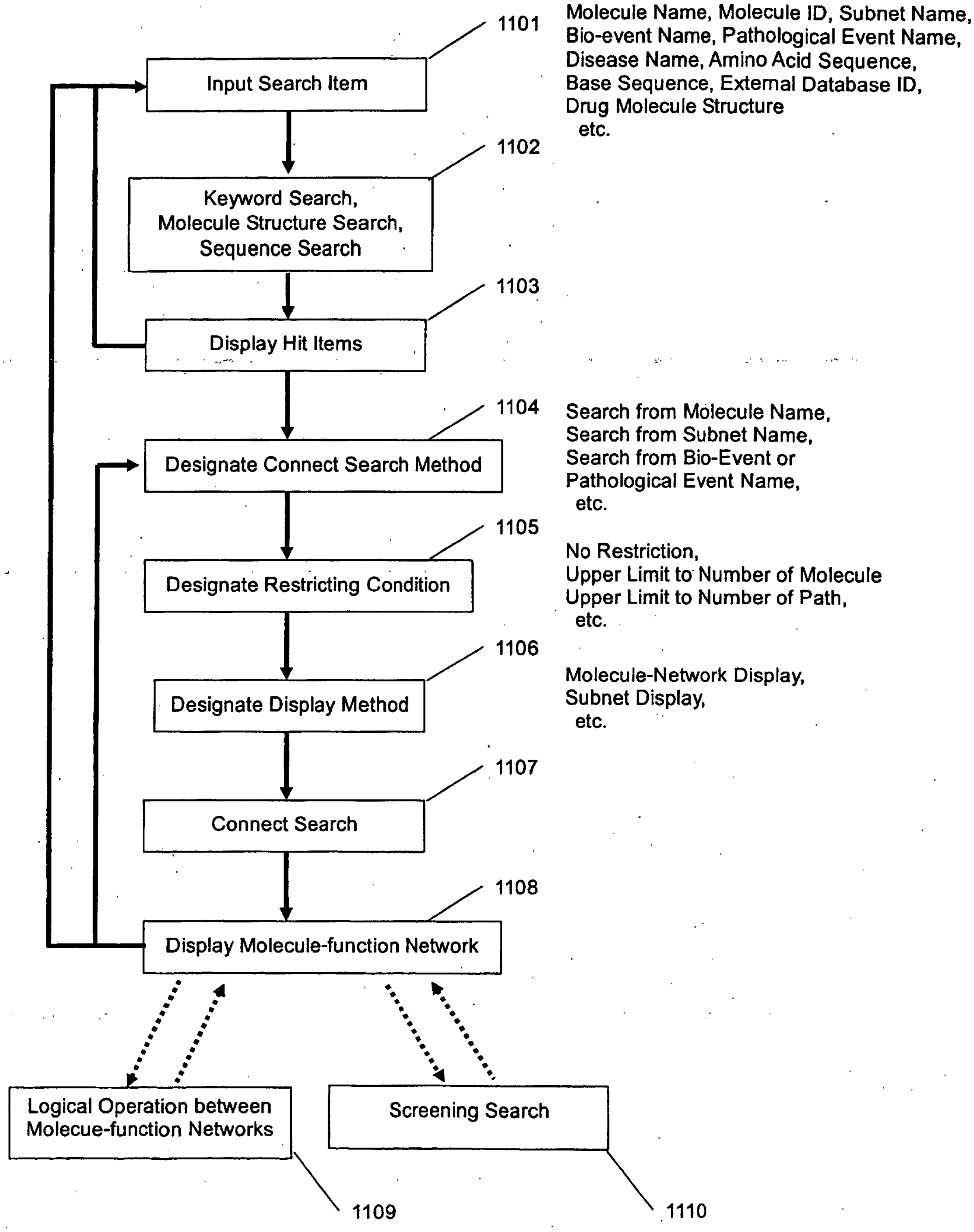
1101
Input Search Item
Molecule Name, Molecule ID, Subnet Name, Bio-event Name, Pathological Event Name, Disease Name, Amino Acid Sequence, Base Sequence, External Database ID, Drug Molecule Structure
etc.
1102
Keyword Search, Molecule Structure Search, Sequence Search
1103
Display Hit Items
1104
Designate Connect Search Method
Search from Molecule Name, Search from Subnet Name, Search from Bio-Event or Pathological Event Name,
etc.
1105
Designate Restricting Condition
No Restriction, Upper Limit to Number of Molecule Upper Limit to Number of Path,
etc.
1106
Designate Display Method
Molecule-Network Display, Subnet Display,
etc.
1107
Connect Search
1108
Display Molecule-function Network
Logical Operation between Molecue-function Networks
Screening Search
1109
1110

Fig.12

| Input Item (Designate 1 Point) |
|---|
| Molecule Name |
| Subnet Name |
| Bio-event Name |

Fig.13

| Input Item (Designate 2 Points) | |
|---|---|
| Molecule Name | Molecule Name |
| Molecule Name | Subnet Name |
| Molecule Name | Bio-event Name |
| Subnet Name | Subnet Name |
| Subnet Name | Molecule Name |
| Subnet Name | Bio-event Name |
| Bio-event Name | Bio-event Name |
| Bio-event Name | Molecule Name |
| Bio-event Name | Subnet Name |

# INTERNATIONAL SEARCH REPORT

International application No.
PCT/JP01/07830

A. CLASSIFICATION OF SUBJECT MATTER
Int.Cl$^7$ G06F17/30, G06F17/60

According to International Patent Classification (IPC) or to both national classification and IPC

B. FIELDS SEARCHED

Minimum documentation searched (classification system followed by classification symbols)
Int.Cl$^7$ G06F17/30, G06F17/60

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2001 |
| Kokai Jitsuyo Shinan Koho | 1971-2001 | Jitsuyo Shinan Toroku Koho | 1996-2001 |

Electronic data base consulted during the international search (name of data base and, where practicable, search terms used)
JICST FILE (JOIS), WPI, INSPEC (DIALOG)

C. DOCUMENTS CONSIDERED TO BE RELEVANT

| Category* | Citation of document, with indication, where appropriate, of the relevant passages | Relevant to claim No. |
|---|---|---|
| X<br>Y | SHIBAGAKI, Y. et al., "A graphical representation system assisting at gene regulatory network construction", Proceedings of the 14th Annual Conference of JSAI, (2000), 03 July, 2000, pages 543 to 544 | 1-3,13,20,21<br>4-12,14-19 |
| Y | GOTO, S. et al., "Organizing and Computing metabolic pathway data in terms of binary relations", Pacific Symposium on Biocomputing, (2nd), Maui, (1996), pages 175 to 186, especially, page 179 | 4-6 |
| Y | HIRAYAMA, "Bunshi Sekkei no tameno Chishiki Data System no Koujika-hou no Kaihatsu; Lead Kagoubutsu Hakken no tameno Keiken-soku no Riron-ka ni kansuru Kenkyuu", Seitai Seigyo Busshitsu no Bunshi Sekkei to Seimitsu Gousei no tameno Kiban Gijutsu no Kaihatsu ni kansuru Kenkyuu (1st period) Seika Houkokusho, Heisei 5-7 nendo (1993-1995) ed., (1996), pages 210 to 221 | 7-12,17-19 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 October, 2001 (04.10.01) | 16 October, 2001 (16.10.01) |
| Name and mailing address of the ISA/<br>Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP01/07830

C (Continuation). DOCUMENTS CONSIDERED TO BE RELEVANT

| Category* | Citation of document, with indication, where appropriate, of the relevant passages | Relevant to claim No. |
|---|---|---|
| Y | OKUBO et al., "Rensai Genome Data Base Keisan-ki ni Kakunou sareta Seimei no Sugata; Dai 6kai, Hatsugen Joho Database to Clustering; Idenshi no Furumai no Hyougen to sore ni motozuku Idenshi no Bunrui", bit, 01 December, 1999, Vol.399, pages 80 to 86 | 14-16 |
| Y | NAKAI, "Genome Kinou Kenkyuu Protocol Micro Array, PCR, Bio-informatics no Saishin Gijutsu kara SNP, Model Seibutsu no Kaiseki made; Chapter 3, Hito Genome Bio-informatics; 1. Genome Informatics", Jikken Igaku separate volume, Post Genome Jidai no Jikken Kouza 1, 10 April, 2000, pages 163 to 167 | 14-16 |
| A | KANEHISA, M., "Pathway Database and Logical Simulation of Genes and Molecules", Genome Science: Hito Genome Kaiseki ni motozuku Bio-Science no Shin-Tenkai, Heisei 8 nendo (1996), No.08283101, March, 1997, pages 218 to 221 | 1-21 |
| A | KANEHISA, M., "Pathway Databases and Higher Order Function", Advances in Protein Chemistry, 06 June, 2000, (received at JICST), Vol.51, pages 381 to 408 | 1-21 |
| PA | JP 2001-188768 A (Japan Science and Technology Corporation), 10 July, 2001 (10.07.01), & WO 01/48690 A1 | 1-21 |

Form PCT/ISA/210 (continuation of second sheet) (July 1992)